Europäisches Patentamt

European Patent Office

Office européen des brevets

(11) Veröffentlichungsnummer: **0 023 882**
**B1**

(12) EUROPÄISCHE PATENTSCHRIFT

(45) Veröffentlichungstag der Patentschrift: **20.01.88**

(51) Int. Cl.⁴: $C\ 12\ N\ 15/00$, $C\ 12\ N\ 9/16$

(21) Anmeldenummer: **80730051.2**

(22) Anmeldetag: **28.07.80**

(54) **Plasmidvektoren, Plasmide mit Genen für alkalische Phosphatasen und Bakterien, die letztere enthalten, ihre Herstellung und Verwendung.**

(30) Priorität: **03.08.79 DE 2931999**

(43) Veröffentlichungstag der Anmeldung:
**11.02.81 Patentblatt 81/06**

(45) Bekanntmachung des Hinweises auf die
Patenterteilung:
**20.01.88 Patentblatt 88/03**

(84) Benannte Vertragsstaaten:
**AT BE CH DE FR GB IT LI LU NL SE**

(56) Entgegenhaltungen:

JOURNAL OF BACTERIOLOGY, Band 120, no.
2, November 1974, M. BRACHA et al.: "Location
of the genes controlling alkaline phosphatase
on the F'13 episome of Escherichia coli", Seiten
970-973

CHEMICAL ABSTRACTS, vol. 90, no. 3, January
16, 1979, Seite 292, Zusammenfassung 18870b,
Columbus, Ohio, US, M.A. NESMEYANOVA et
al.: "Study of interrelation of metabolic and
genetic regulation of alkaline and acid
phosphatases in Escherichia coli cells"

(73) Patentinhaber: **SCHERING
AKTIENGESELLSCHAFT Berlin und Bergkamen
Müllerstrasse 170/178 Postfach 65 03 11
D-1000 Berlin 65 (DE)**

(72) Erfinder: **Siewert, Gerhard, Dr.
Im Fischgrund 50c
D-1000 Berlin 28 (DE)**
Erfinder: **Boidol, Werner
Schaperstrasse 19
D-1000 Berlin 15 (DE)**
Erfinder: **Daum, Joachim, Dr.
Kyllmannstrasse 22d
D-1000 Berlin 45 (DE)**

(56) References cited:
AGRIC. BIOL. CHEM. Band 44, Nr. 5, Mai 1980,
K. YODA et al.: "Cloning of the structural gene
of alkaline phosphatase of Escherichia coli
K12", Seiten 1213-14

CHEMICAL ABSTRACTS, Band 88, Nr. 21, 22.
Mai 1978, Seiten 286, Zusammenfassung Nr.
1487583, Columbus, Ohio, US, M.A.
NESMEYANOVA et al.: "Metabolic and genetic
control of isoenzyme spectrum of alkaline
phosphatase in Escherichia coli"

EP 0 023 882 B1

Courier Press, Leamington Spa, England.

0 023 882

(56) References cited:

CHEMICAL ABSTRACTS, Band 77, Nr. 3, 17. Juli
1972, Seite 211, Zusammenfassung 15915r,
Columbus, Ohio, US, D.A. LENNETTE:
"Characterization of the defects in an
Escherichia coli alkaline phosphatase mutant"

CHEMICAL ABSTRACTS, Band 81, Nr. 19, 11.
November 1974, Seite 247, Zusammenfassung
Nr. 116999j, Columbus, Ohio, US, H. MORRIS et
al.: "Pleiotropic effects of mutations involved in
the regulation of Escherichia coli K-12 alkaline
phosphatase"

CHEMICAL ABSTRACTS, Band 89, Nr. 9, 28.
August 1978, Seite 214, Zusammenfassung
72856u, Columbus, Ohio, US, A. NAKATA et
al.: "Escherichia coli mutants deficient in the
production of alkaline phosphatase isozymes"

**Beschreibung**

Verfahren zur Isolierung von alkalischen Phosphatasen aus Bakterien sind bereits aus der Monographie "The Enzymes, Ed. P. D. Boyer, Acad. Press 1971, Vol. IV. S. 373—415" bekannt.

Es ist ein Plasmid mit einem E. coli-Phosphatasegen bekannt, bei dem es sich um einen F'-Faktor handelt, der durch in-vivo-Rekombination eines Fertilitätsfaktors mit einem Abschnitt chromosomaler DNS in E. coli entstanden ist [M Bracha, E. Yagil, J. Bacteriol, *120*, 970—973 (1974)]. Die Isolierung eines solchen Plasmids ist bisher nur für das E. coli-Phosphatasegen beschrieben worden und stellt kein allgemein anwendbares Verfahren zur Klonierung von Phosphatasegenen dar. Insbesondere ist es auf diese Weise nicht möglich, Gene ganz unterschiedlichen Ursprungs, z.B. aus niederen Eukaryonten, zu klonieren. Das F'13-Plasmid liegt wie viele andere Fertilitätsfaktoren nur in 1—2 Kopien pro Zelle vor, wodurch auch nur eine geringe Stimulierung der Phosphatasebildung bewirkt wird.

Alkalische Phosphatasen (EC 3.1.3.1) sind Enzyme, welche von einer Vielzahl prokaryotischer und eukaryotischer Organismen produziert werden und welche die unterschiedlichsten Phosphorsäuremonoester zu anorganischem Phosphat und dem entsprechenden Alkohol hydrolysieren können [Zusammenfassung von T. W. Reid und I. B. Wilson in The Enzymes, Ed. P. D. Boyer, Acad, Press 1971, Vol. IV. S. 373—415]. Sie werden in der biochemischen und molekularbiologischen Forschung sowie in der klinischen Diagnostik als präparative und analytische Reagenzien eingesetzt.

In der DOS 26 17 350 wird z.B. eine trägerfixierte alk. Phosphatase bei der Herstellung von definierten Oligonukleotiden beschrieben. Eine quantitative Bestimmung von Phosphorsäuremonoestern ist möglich durch Messung des bei der Hydrolyse mit alk. Phosphatase gebildeten anorganischen Phosphats [Acta Chem. Scand. B*31*, 125—129 (1977)]. Eine wichtige Anwendung ist der Einsatz als leicht nachweisbares Markierungsenzym bei verschiedenen Enzym-Immunotests, wobei die alk. Phosphatase je nach Art der Bestimmung sowohl an ein Antigen als auch an Antikörper gekoppelt wird. Enzym-Immunotests erlauren eine spezifische und quantitative Bestimmung zahlreicher Substanzen (Hormone, Virus-Proteine, verschiedene Zellwandantigene, Antikörper usw.) in kleinsten Mengen (E. Engvall, A. J. Pesce, ed., Quantitative Enzym Immunoassay, Blackwell Scientific Publications, Oxford 1978).

Für viele dieser Anwendungen wird bevorzugt die alk. Phosphatase aus E. coli eingesetzt, wobei u.a. die hohe Temperaturstabilität dieses Enzyms (20 Minuten bei 85°C) verglichen mit Säugetierphosphatasen von Vorteil ist (Jap.-PS J5 2099—211).

Manche Bakterien, insbesondere E. coli, synthetisieren einzelne Enzyme in stark erhöhten Mengen, wenn von dem entsprechenden Gen, welches normalerweise in Wildtypbakterien nur einmal pro Zelle vorhanden ist, zahlreiche Kopien pro Zelle vorliegen [I. G. Young et al., Gene *4*, 25—36 (1978)]. Diese hohe Genkopienzahl (Ca. 10—20 Kopien pro Zelle) wird dadurch erreicht, daß DNS-Fragmente, welche das betreffende Gen tragen, mit Hilfe der Techniken zur in-vitro-Neukombination von DNS selektiv auf Bakterien übertragen werden.

Verschiedene Verfahren zur gezielten Übertragung von Genen auf Bakterien, welche auch als DNS-Klonierung bezeichnet werden, sind in K. N. Timmis, S. N. Cohen, F. C. Caballo, in Progress in Molecular and Subcellular Biology, F. E. Hahn, ed., Vol. 6, S. 1—58, Springer Verlag 1978, beschrieben worden, Sie beruhen alle darauf, daß ein DNS-Fragment, welches das entsprechende Gen trägt, mit einer Vektor-DNS verknüpft und in dieser Form in eine geeignete Empfängerzelle eingeführt und darin als neukombinierte DNS repliziert wird. Als Vektoren dienen Plasmide oder Bakteriophagen. Plasmide sind ringförmige doppelsträngige DNS-Moleküle, welche in manchen Bakterienstämmen gefunden werden und sich darin unabhängig von der chromosomalen DNS (extrachromosomal) replizieren können. Ein wesentliches Hilfsmittel bei der Klonierung von DNS sind die Restriktionsendonukleasen, welche doppelsträngige DNS-Moleküle an genau definierten Stellen zu kleineren Fragmenten zer schneiden können.

Ein häufig angewandtes Verfahren zur Klonierung von DNS-Fragmenten, welche Gene für Stoffwechselenzyme tragen, besteht z.B. darin, daß chromosomale DNS eines geeigneten Spenderorganismus und Plasmid-DNS mit einer passenden Restriktionsendonuklease und nach Vermischung mit DNS-Ligase umgesetzt werden, wobei aus den DNS-Fragmenten eine Vielzahl verschiedener linearer und zirkulärer oligomerer Produkte entsteht, u.a. auch Ringmoleküle, welche pro Plasmid-Molekül ein Molekül des DNS-Fragments mit dem gesuchten Gen enthalten. Die DNS-Mischung wird dann in einem als Transformation bezeichneten Prozeß auf geeignete Empfängerbakterien übertragen, aus der gesamten Zellpopulation werden diejenigen Zellen selektioniert, welche die gesuchte neukominierte Plasmid-DNS enthalten. Varianten diese Verfahrens beruhen z.B. darauf, daß die Fragmentierung der chromosomalen DNS durch Scherkräfte (Ultraschall etc.) bewirkt wird und daß die Kopplung an das Plasmid über homopolymere DNS-Einzelstrangenden erfolgt, welche mit dem Enzym Terminale Transferase erzeugt wurden [L. Clarke und J. Carbon, Proc. Nat. Acad. Sci. (USA) *72*, 4361—4365 (1975)]. Anstelle von Plasmiden ist auch Phagen-DNS erfolgreich zur Klonierung verwendet worden.

Bei der Neukombination von DNS, insbesondere wenn von einer komplexen Mischung chromosomaler DNS-Fragmente ausgegangen wird, entsteht in vitro primär einer große Zahl verschiedener Hybridmoleküle. Zur Klonierung von definierten DNS-Fragmenten benötigt man daher ein Selektionsverfahren, welches nach der Transformation bzw. Transfektion die Isolierung von Zellklonen mit der gesuchten neukombinierten DNS ermöglicht. Trägt das zu klonierende Fragment ein im Empfängerorganismus exprimierbares Stoffwechselgen, so verwendet man als Empfänger gut transformierbare Mutanten, in denen das betreffende Gen inaktiviert ist und die das entsprechende Stoffwechselmerkmal erste dann wieder aufweisen, wenn sie das Gen durch Transformation mit neukombinierter DNS in replizierbarer und damit vererbbarer Form aufgenommen haben.

Zunächst wird unter Verwendung eines an sich bekannten Mutationsverfahrens von einem gut transformierbaren E. coli-Stamm, E. coli K12 HB101, eine bisher nicht bekannte Mutante hergestellt, in welcher das Strukturgen für alkalische Phosphatase (phoA) inaktiviert ist. Diese Mutante wird unter Anwendung der oben genannten Verfahren der DNS-Klonierung zur Transformation mit in-vitro neukombinierter DNS und zur Selektion von Zellklonen eingesetzt, die neukombinierte DNS mit Genen für alkalische Phosphatase enthalten. Bei den in J. Bacteriol. *119*, 583—592 (1974) beschriebenen phosphatasenegativen Mutanten von E. coli handelt es sich dagegen um Stämme, die wegen der noch vorhandenen Fähigkeit zur in-vivo-Restriktion und -Rekombination schlecht transformierbar und daher zur Klonierung von Phosphatasegenen ungeeignet sind.

Das erwähnte Mutationsverfahren arbeitet mit N-Nitrosoverbindungen, wie sie für Mutationen üblicherweise verwendet werden. Als N-Nitrosoverbindungen kommen z.B. Nitrosoharnstoffe und -guanidine in Betracht. Als besonders geeignete Verbindung erweist sich 1-Nitroso-3-nitro-1-methylguanidin.

Der Gegenstand der Erfindung ist in den Ansprüchen definiert.

Die erfindungsgemäßen phoA-Gene enthaltenden Plasmide können hergestellt werden, indem man chromosomale DNS aus Wildtyp-Bakterien und eine Antibiotika-resistentes Plasmid mit Restriktionsendonukleasen, für die das Plasmid Schnittstellen besitzt, umsetzt und nach anschließender Verknüpfung der Schnittstellen mit DNS-Ligase unter Zuhilfenahme der aus E. coli K12 HBlol erhaltenen phoA-Mutante E. coli SB44 nach dem oben beschriebenen Verfahren Plasmide mit Strukturgenen für alkalische Phosphatase isoliert. Aus diesen Plasmiden lassen sich weitere, ebenfalls phoA-Gene enthaltene Plasmide herstellen. Dazu schneidet man aus ihnen mit Hilfe von Restriktionsenzymen Subfragmente heraus, die das phoA-Gen enthalten, und rekombiniert diese Subfragmente mit Antibiotika-resistenten Vektorplasmiden in Gegenwart von DNS-Ligase.

Unter Wildtypbakterien werden solche Bakterien verstanden, die vom Fachmann zu mikrobiologischen Arbeiten üblicherweise verwendet werden. Als besonders geeignet haben sich Bakterien der Wildtypstämme E. coli K12 und Serratia marcescens erwiesen.

Erfindungsgemäße Plasmide werden erhalten, indem man z.B. die Gesamt-DNS aus E. coli K12 Wildtypbakterien und das Plasmid pBR313, welches Resistenz gegen Ampicillin vermittelt, mit der Restriktionsendonuklease BamHI schneidet und mit DNS-Ligase neukombiniert. Die oben erwähnte phosphatasenegative Mutante (E. coli SB44) wird mit der ligasebehandelten DNS-Mischung unter Transformationsbedingungen inkubiert, wobei ein kleiner Teil der Zellen (ca. 1 aus $10^4$) Plasmid-DNS aufnimmt. Durch Vermehrung in einem Ampicillin-enthaltenen Medium, in welchem die ampicillinsensitive Mutante nicht wachsen kann, werden die transformierten Zellen angereichert. Nach Ausplattieren der angereicherten Zellpopulation auf Agarplatten mit sehr niedrigem Phosphatgehalt und Inkubation bei 37°, wobei die Phosphatasebildung maximal induziert wird, werden die so erhaltenen Einzelkolonien mit einer Lösung von p-Nitrophenylphosphat überschichtet. Phosphatasepositive Kolonien hydrolysieren diese Substanz zu gelbem p-Nitrophenol [ K. Kreuzer et al., Genetics *81*, 459—468 (1975)]. Im Mittel werden unter ca. 10.000 farblosen eine gelbe Kolonie gefunden. Aus diesen pho$^+$-Kolonien wird nach der Methode von G. O. Humphreys et al. Biochem. Biophys, Acta *383*, 457—463 (1985) Plasmid-DNS isoliert. Die Analyse des dabei erhaltenen und als pSB47 bezeichneten Plasmids durch Spaltung mit BamHI und Gelelektrophorese der Spaltprodukte zeigte, daß es aus dem Ausgangsplasmid pBR313 und einem zusätzlichen DNS-Fragment (MG ca. 11 Md) besteht. Der Nachweis, daß pSB47 ein Phosphatasegen enthält, wird durch erneute Transformation der Phosphatasemutante E. coli SB44 mit dem Plasmid erbracht. Dabei zeigte sich, daß alle ampicillinresistenten Kolonien auch phosphatasepositiv waren. Da pBR313 kein Gen für alk. Phosphatase hat, muß sich dieses auf dem zusätzlichen DNS-Fragment vom MG 11 Md befinden.

Die Herstellung des Plasmids pSB51 erfolgt auf ganz ähnliche Weise wie die von pSB47 nur mit dem Unterschied, daß chromosomale DNS aus Serratia marcescens anstelle von E. coli-DNS eingesetzt wurde, daß als Vektor pWB2 verwendet wurde und daß sowohl die chromosomale DNS als auch der Vektor mit der Restriktionsendonuklease HindIII geschnitten wurde. pSB51 enthält neben dem Vektor pWB2 ein DNS-Fragment vom MG 2,5 Md, auf welchem ähnlich wie oben ein Phosphatasegen nachgewiesen wurde. Darüber hinaus zeigt der Vergleich mit authentischen Phosphatasen aus E. coli und S. marcescens durch Disk-Elektrophorese, daß die E. coli-Zellen mit dem Plasmid pSB51 ein mit S. marcescens-Phosphatase identisches Enzym produzieren. Dies beweist, daß das DNS-Fragment von MG 2,5 Md in pSB51 aus Serratia-DNS stammt und ein Serratia-Phosphatasegen trägt.

Auch eine nachträgliche Modifikation der Plasmide ist in manchen Fällen möglich, ohne daß wesentliche Eigenschaften verloren gehen. So läßt sich z.B. das Fragment mit dem MG 11 Md in pSB47 durch Spaltung mit der Restriktionsendonuklease HindIII in mehrere Teilfragmente zerlegen, von denen das zweitgröße Fragment (MG 3,1 Md) das Phosphatasegen trägt. Dieses wurde durch Ligaseverknüpfung mit anschließender Transformation und Selektion wie oben an einen von pBR322 [F. Bolivar et al., Gene *2*, 95—113 (1977)] abgeleiteten Vektor (pSB18) gekoppelt, dem die EcoRI-Schnittselle des pBR322 fehlt. Aus

4

**0 023 882**

diesem läßt sich durch Umsetzung mit HindIII und BamHI ein für die Replikation und Selektion unnötiges Teilfragment von MG 0,18 Md entfernen und durch das Subfragment mit dem Phosphatasegen aus pSB47 ersetzen. Es resultiert das Phosphatase-Plasmid pSB53.

Auf ähnliche Weise wie das Plasmid pSB47 läßt sich ein weiteres Plasmid, pSB50, herstellen. Der Unterschied liegt lediglich darin, daß als Ausgangsvektor pWB2 verwendet wird, der zusammen mit chromosolamer DNS aus E. coli mit der Restriktionsendonuklease HindIII umgesetzt wird. Bakterienstämme mit dem so erhaltenen Plasmid pSB50 produzieren ebenso wie die mit pSB47 alkalische Phosphatase.

Gegenstand der Erfindung ist auch ein Verfahren zur Herstellung neuer Bakterienstämme, das darin besteht, daß man kompetente Zellen des betreffenden Bakterienstammes mit den Phosphataseplasmiden unter Transformationsbedingungen in an sich bekannter Weise inkubiert und aus der erhaltenen Zellpopulation die Antibiotikaresistenten Zellen isoliert. Mit den so gewonnenen Bakterienstämmen, die in ihren Plasmiden Gene für alkalische Phosphatase enthalten, lassen sich größere Mengen an alkalischer Phosphatase herstellen als das bisher möglich war. Dazu werden die Stämme in einem Medium mit minimalen Phosphatgehalt fermentiert und die gebildete Phosphatase nach an sich bekannten Verfahren isoliert. Es gelingt z.B. die alkalische Phosphatase der Serratia marcescens durch E. coli SB44 produzieren zu lassen.

In der Tabelle sind die Konzentrationen an alkalischer Phosphatase zusammengefaßt, ausgedrückt in Einheiten pro ml Kulturflüssigkeit, welche für verschiedene Stämme mit Phosphataseplasmiden sowie die entsprechenden Kontrollstämme ohne oder mit chromosomalen Phosphatasegen ermittelt wurden, und zwar unter Bedingungen, unter denen die Bildung von alkalischer Phosphatase möglichst hoch stimuliert ist (Induktion durch geringen Phosphatgehalt des Mediums). In allen Fällen zeigte sich, daß die Anwesenheit eines der Phosphataseplasmide eine signifikante Erhöhung des Gehalts an Phosphatase bewirkte, und zwar bis zum ilfachen der Werte für die Stämme, die nur eine chromosomales Phosphatasegen haben. Dies gilt insbesondere auch für Serratia marcescens als Beispiel einer Wirtszelle, die E. coli-Plasmide aufnehmen kann.

Das aus J. Bacteriol. *120*, 970—973 (1974) bekannte E. coli-phosphatasegen-hastige Plasmid liegt nur in 1—2 Kopien, die erfindungsgemäßen Plasmide dagegen in bis zu 20 Kopien pro Zelle vor.

TABELLE:
Phosphataseproduktion von Bakterien mit phoA-Plasmiden‡

| Wirtzelle | Plasmid | Herkunft des phoA-Gens im Plasmid | Phosphatase-Aktivität |
|---|---|---|---|
| E. coli SB44 (phoA-Mutante) | — | — | 0 |
| | pSB47 | E. coli | 11,0 |
| | pSB50 | E. coli | 12,5 |
| | pSB53 | E. coli | 20,0 |
| | pSB51 | S. marcescens | 19,5 |
| | pSB60 | E. coli | 20,1 |
| E. coli HB101 | pWB2 | (Vektor ohne phoA-Gen) | 5,0* |
| | pSB47 | E. coli | 18,0 |
| | pSB50 | E. coli | 13,0 |
| E. coli K12 (Wildtyp) | — | — | 2,5 |
| | pSB47 | E. coli | 11,0 |
| | pSB50 | E. coli | 13,6 |
| | pSB51 | S. marcescens | 19,5 |
| S. marcescens (Wildtyp) | — | — | 4,1 |
| | pSB51 | S. marcescens | 44,0 |
| | pSB53 | E. coli | 19,6 |

*E. coli HB101 ohne oder mit anderen Vektoren hat die gleiche Phosphataseaktivität.

‡) LP-Medium [K. Kreuzer et al., Genetics *81*, 459—468 (1975)], das mit 0,2% Casamino Acids supplementiert worden war, wurde mit 5% einer ausgewachsenen Kultur des jeweiligen plasmidhaltigen Stammes beimpft und 23 Stunden bei 37° geschüttelt ($E_{500}$=7,0). Danach wurde 1 ml Kulturflüssigkeit mit 0,01 ml Toluol 20 Minuten bei 37° geschüttelt. In der toluolbehandelten Zellsuspension wurde alkalische Phosphatase nach Kreuzer et al. bestimmt. Die Phosphataseaktivität ist in Einheiten pro ml Kulturflüssigkeit angegeben. Eine Einheit ist die Enzymmenge, die unter Testbedingungen ein Extinktionszunahme $E_{410}$=1,0 pro Minute bewirkt.

5

Die erfindungsgemäßen Plasmide können außerdem besonders vorteilhaft als Plasmidvektoren zur Expression eukaryotischer Proteine in Escherichia coli und anderen Bakterien durch Bildung von Fusionsproteinen mit N-terminalen Teilsequenzen aus alkalischen Phosphatasen benutzt werden. Worin ihr Vorteil gegenüber anderen Plasmidvektoren liegt, soll nachstehend noch näher erläutert werden.

Die Verfahren zur in vitro-Neukombination von DNS unter Verwendung von Plasmid- oder Phagenvektoren und die Übertragung der dabei entstehenden Hybrid-Moleküle auf geeignete Wirtszellen durch Transformation oder Transfektion haben es ermöglicht, auch DNS aus höheren eukaryotischen Zellen in replikationsfähiger Form auf Bakterien zu übertragen (K. N. Timmis in Progress in Molecular and Subcellular Biology, F. E. Hahn, ed., Vol. 6, S. 1—58, Springer Verlag 1978). Jedoch waren die so transformierten Bakterienzellen in der Regel nicht in der Lage, die Proteine, welche den auf der übertragenen eukaryotischen DNS vorhandenen Genen entsprachen, zu synthetisieren.

Die Expression von Genen aus höheren eukaryotischen Zellen in Bakterien gelingt erste durch Verwendung von speziellen Plasmidvektoren, die so konstruiert sind, daß das eukaryotische Gen in ein auf dem Plasmid vorhandenes bakterielles Gen integriert werden kann, das für sich allein die Synthese eines geeigneten, gut charakterisierten bakteriellen Proteins bewirkt. Ist die eukaryotische DNS mit der richtigen Orientierung und im korrekten Triplettraster integriert, produzieren die so ausgestatteten Bakterienzellen, die mit einem derartigen Hybrid-Molekül transformiert werden, ein Fusionsprotein, das vom Aminoende her mit einem Teil der bakteriellen Aminosäure-Sequenz beginnt und sich zum Carboxylende hin mit der Aminosäure-Sequenz fortsetzt, die der integrierten eukaryotischen DNS entspricht. Aus dem Fusionsprotein kann dann mit geeigneten enzymatischen oder proteinchemischen Methoden, z.B. durch Spaltung eines an der Verknüpfungsstelle vorhandenen Methionins mit Bromcyan, das eukaryotische Protein gewonnen werden.

Das erste Beispiel einer derartigen Polypeptid-Synthese war die mikrobielle Herstellung des Peptidhormons Somatostatin (14 Aminosäuren), das in Form eines Fusionsproteins mit dem bakteriellen (Enzym ß-Galactosidase erhalten wurde [K. Itakura et al., Science *198*, 1056—1063 (1977)]. Auf ähnliche Weise gelang kürzlich die mikrobielle Synthese der A- und B-Ketten von Humaninsulin, wobei wie im Falle des Somatostatins vollsynthetischse DNS-Sequenzen an ein Gen für ß-Galactosidase gekoppelt wurden (DE—OS 28 48 051, 28 48 052, 28 48 053). Weitere Beispiele sind die Herstellung von Bakterienstämmen, die Ratten-Proinsulin in Form eines Fusionsproteins mit dem bakteriellen Enzym ß-Lactamase [L. Villa-Komaroff et al., Proc. Natl. Acad. Sci. USA *75*, 3727—3731 (1978)] und ein Ovalbumin-ähnliches Protein mit Sequenzen der ß-Galactosidase [ O. Mercereau-Puijalon et al., Nature *275*, 505—510 (1978)] produzieren. Diese Methode der mikrobiellen Polypeptid-Synthese sollte auch auf die Herstellung anderer eukaryotischer Peptide und Proteine anwendbar sein, die auf andere Weise zum Teil nur sehr schwer zugänglich sind, wie z.B. menschliches Wachstumshormon, Interferon, Virusproteine zur aktiven Immunisierung, spezifische Antikörper usw.

Der Erfolg mikrobieller Polypeptid-Synthesen unter Verwendung von neukombinierter DNS hängt ganz wesentlich von den Eigenschaften der verwendeten Vektoren ab. In den oben genannten Beispielen wurden Plasmidvektoren verwendet, bei denen die Fremd-DNS in Gene für ß-Galactosidase oder ß-Lactamase integriert wurde. Diese beiden bakteriellen Proteine haben als Fusionspartner für die Expression von Fremd-DNS jedoch auch Nachteile. Fusionsproteine mit ß-Galactosidase lassen sich wie das Originalenzym selbst durch entsprechende Medienzusammensetzung (Induktion mit ß-Galactosiden) in relativ großen Mengen produzieren ($10^5$ Moleküle pro Zelle), die Proteine werden jedoch von den Zellen z.B. nicht ins Medium ausgeschieden und sind daher nur durch Zellaufschluß zu isolieren. ß-Lactamase und die davon abgeleiteten Fusionsproteine werden bei der Synthese durch die Zellmembran in den periplasmatischen Raum transportiert, was eine einfache Isolierung ermöglicht (kein (Zellaufschluß), hier wiederum ist die Ausbeute nur gering (ca. 100 Moleküle pro Zelle).

Die vorliegende Erfindung betrifft die Herstellung von Plasmidvektoren, die den Einbau von Fremd-DNS in alkalische-Phosphatase-Gene ermöglichen. Zellen, die mit aus solchen Plasmidvektoren gebildeten Hybridplasmiden oder -phagen transformiert werden, stellen dann Fusionsproteine her, deren Aminosäuresequenz durch die Nukleotidsequenz sowohl des Gens für alkalische Phosphatase als auch der integrierten Fremd-DNS bestimmt wird, Daher stellt die Anwendung dieser Vektoren eine Verbesserung im Vergleich zu den oben zitierten Verfahren der mikrobiellen Synthese von eukaryotischen Peptiden und Proteinen dar.

Alkalische Phosphatasen vereinigen als Fusionspartner bei der Expression eukaryotischer Gene in sich die Vorzüge von ß-Galactosidase und ß-Lactamase. Sie lassen sich wie ß-Galactosidase durch entsprechende Medienzusammensetzung (niedrige Phosphatkonzentrations) hoch induzieren und werden wie ß-Lactamase durch die Zellmembran in den periplasmatischen Raum transportiert (Zusammenfassung T. W. Reid, I. B. Wilson in The Enzymes, Ed. P. D. Boyer, Acad. Press 1971, Vol. IV, S. 373—415).

Damit Phosphatase-Vektoren in der oben beschriebenen Weise zur Herstellung von Fusionsproteinen verwendet werden können, müssen sie so konstruiert sein, daß die Restriktionssequenz, über welche die zu exprimierende Fremd-DNS integriert werden soll, außerhalb des Phosphatasegens in dem übrigen Teil des Vektors möglichst nicht noch einmal vorhanden ist. Die in den Beispielen 4 und 6 beschriebenen Plasmide pSB53 und pSB86 erfüllen diese Bedingungen.

Es wurde bereits gezeigt, daß sich das Gen für alkalische Phosphatase aus E. coli K12 in dem Plasmid pSB47 auf einem DNS-Fragment vom Molekulargewicht (4600Bp befindet, welches durch gleichzeitige Spaltung mit den Restriktionsendonukleasen BamHI und HindIII freigesetzt werden kann. Dieses Fragment wurde durch Umsetzung mit DNS-Ligase an das Plasmid pSB18 gekoppelt, welches aus pBR322 durch Beseitigung der EcoRI-Sequenz hergestellt worden war und Resistenz gegen Ampicillin und Tetracyclin bewirkt. pBS18 wurde vor der Ligase-Reaktion ebenfalls mit HindIII und BamHI geschnitten, wobei ein kleines Teilfragment entfernt wird, welches für die Tetracylin-Resistenz mitverantwortlich ist. Nach Transformation der Phosphatase-negativen Mutante SB44 mit der DNS-Mischung wurden Zellen selektioniert, welche Ampicillin-resistent, Tetracyclin-sensitiv und Phosphatase-positiv waren. Aus diesen ließ sich das Plasmid pSB53 isolieren. Das darin enthaltene phoA-Fragment kann auch direkt aus chromosomaler DNS aus E. coli K12 durch Spaltung mit HindIII und BamHI erhalten werden. Wesentliche Voraussetzung für die Isolierung von pSB53 und der übrigen Vektoren ist die Verwendung der Phosphatase-negativen Mutante E. coli SB44.

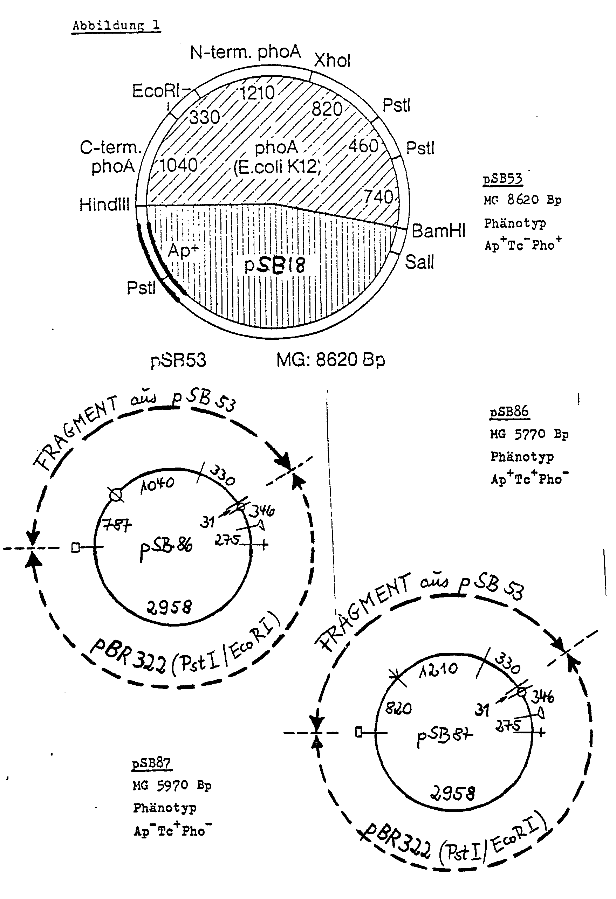

Die Restriktionskarte von pSB53 für die Enzyme HindIII, BamHI, EcoRI, PstI, XhoI und SalI ist in Abb. 1 dargestellt. Es ließ sich zeigen, daß durch Austausch des EcoRI-Fragments von 330Bp in pSB53 gegen ein anderes EcoRI-Fragment das Gen für alkalische Phosphatase inaktiviert wird, Diese Gen muß daher im Bereich der EcoRI-Restriktionssequenzen liegen. Durch partielle Spaltung von pSB53 wurden zwei Fragmente mit den Molekulargewichten 2157 und 2360Bp erhalten, von denen das eine (2157Bp) die in Abb. 1 dargestellten Sequenzen mit 787,1040 und 330Bp umfaßt, während das andere (2360Bp) aus den Sequenzen mit 330, 1210 und 820Bp besteht. Die beiden Fragmente wurden mit Hilfe von pBR322 in E. coli SB44 kloniert, wobei die in Abb. 1 dargestellten Plasmide pSB86 und pSB87 resultierten. Beide Plasmide haben kein intaktes Gen für alkalische Phosphatase mehr, woraus gefolgert werden muß, daß beide auf pSB53 vorhandenen EcoRI-Restriktionssequenzen in diesen Gen liegen. Die PstI-Sequenzen in pSB53 liegen außerhalb des phoA-Gens.

Es ist gezeigt worden, daß die Beeinflussung der Phosphatase-Bildung durch die Phosphat-Konzentration des Mediums ein positiv kontrollierter Prozeß ist und daß für die Transkription des phoA-Gens ein Aktivator erforderlich ist, der an eine Promotor-Region dieses Gens gebunden wird. Ein wesentlicher Bestandteil des Aktivators ist das Produkt des phoB-Gens (H. Morris et al., J. Bacteriol. *119*, 583—592 (1974); C. Pratt und A. Torriani, Genetics *85*, 203—208 (1977)).

Eines der beiden Plasmide pSB86 und pSB87, die beide kein vollständiges Phosphatase-Gen mehr besitzen, sollte die Bindungssequenz für den Aktivator und damit auch den Genbereich enthalten, der dem N-terminalen Ende der alkalischen Phosphatase entspricht. In einem Stamm mit einem intakten chromosomalen phoA-Gen sollte dieses Plasmid die Phosphatase-Bildung reduzieren, da es mit dem chromosomalen Gen um den Aktivator konkurriert und diesen teilweise bindet.

Wir haben nun gefunden, daß der mit pSB87 transformierte Stamm E. coli HB101 die gleiche Menge Phosphatase bildet wie E. coli HB101 ohne Plasmid (je 5,6 Einheiten/ml Kulturflüssigkeit; gemessen unter den gleichen Bedingungen wie auf S. 13 beschrieben), während E. coli HB101 mit pSB86 nur ca 25—30% dieser Menge (1,5 E/ml) produziert. Daraus ist zu folgern, daß sich die Aktivator-Bindungssequenz und damit auch der Transkriptionsbeginn des phoA-Gens auf pSB86 befinden. Die Transkriptionsrichtung des phoA-Gens auf pSB53 weist daher von der HindIII- zur BamHI-Sequenz (Uhrzeigersinn in Abb. 1); das Gen beginnt mit dem Ende, welches dem Aminoende der Phosphatase entspricht, links von dem EcoRI-Fragment mit 330Bp, zwischen der EcoRI und der HindIII-Sequenz und erstreckt sich über die beiden EcoRI-Sequenzen in das XhoI/EcoRI-Fragment mit 121o Bp.

Austausch des EcoRI-Fragments mit 330Mp in pSB53 gegen ein anderes DNS-Fragment ermöglicht daher bei richtiger Orienterung und korrekten Triplettraster die Herstellung von Fusionsproteinen, welche vom Aminoende her mit einer Teilsequenz der alkalischen Phosphatase aus E. coli beginnen.

Ein anderer Vektor, der die Herstellung der gleichen Fusionsproteine wie pSB53 ermöglicht, ist das Plasmid pSB86 (Abb. 1), das noch den aminoständigen Teil des phoA-Gens enthält. Auch Plasmide, die nur noch das EcoRI/HindIII-Fragment (1040) aus pSB53 enthalten, sollten den gleichen Zweck erfüllen. Aus pSB53 oder pSB47 könnte man Pst-Fragmente mit dem gesamten Pho-A-Gen erhalten und zur Konstruktion neuer Vektoren verwenden, um nur einige der zahlreichen Kombinationsmöglichkeiten zu nennen. Durch Prüfung weiterer Restriktionsendonuklease ließen sich wahrscheinlich andere Verknüpfungsstellen für die Herstellung fusionierter Gene mit Teilsequenzen des Gens für alkalische Phosphatase finden.

Weitere Möglichkeiten zur Konstruktion von Vektoren für die Bildung von Hybridgenen und Fusionsproteinen (Expressionsvektoren) ergeben sich durch nachträgliche Einführung von Restriktionssequenzen in das Gen für alkalische Phosphatase. Verschiedene Verfahren, die dies ermöglichen sollten, sind beschrieben worden. So wurde z.B. die Bildung von EcoRI-Sequenzen in einem Phagenvektor durch in vitro-Mutation nach Methylierung erreicht (B. Gronenborn und I. Messing, Nature *272*, 375—377 (1978)).

Von sehr viel breiterer Anwendbarkeit ist sin von F. Heffron, M. So und B. I. McCarthy (Proc. Natl. Acad. Sci. USA *75*, S. 6012—6016, 1978) beschriebenes Verfahren, bei welchem ein synthetisches Oligonukleotid, welches die gewünschte Restriktionssequenz enthält, in ein auf einem Plasmid-Vektor befindliches Gen eingefügt wird. Dazu wird die Vektor-DNS mit einer geeigneten Endonuklease, welche die ringförmige Doppelstrang-DNS an vielen verschiedenen Stellen spalten kann, unter Bedingungen unvollständiger Spaltung umgesetzt, so daß zu einem großen Teil lineare Moleküle voller Länge entstehen. Diese werden durch ein geeignetes Verfahren, z.B. präparative Gelelektrophorese, von den übrigen Reaktionsprodukten

7

abgetrennt, evtl. vorhandene Einzelstrangenden werden mit Hilfe von DNS-Polymerase in komplette Doppelstrangenden überführt. An die Enden der DNS wird mit T4-Ligase die synthetische Restriktionssequenz gekoppelt. Nach Schneiden mit der entsprechenden Restriktionsendonuklease zur Erzeugung von komplementären Einzelstrangenden wird zwecks Ringschluß erneut mit DNS-Ligase umgesetzt. Dabei entstehen Ringmoleküle, welche die synthetische Restriktionssequenz an all den Stellen enthalten könne, an denen die Endonuklease zuvor gespalten hat. Durch Transformation einer geeigneten Wirtszelle, in diesem Fall der phoA-Mutante E. coli SB44, und Selektion von Transformanten, die nicht positive für das betreffende Gen geworden, d.h. in diesem Fall pho⁻ geblieben sind, erhält man Zellkone, aus denen sich Plasmide isolieren lassen, die die synthetische Restriktionssequenz an verschiedenen definierten Stellen in das Plasmid-Gen (hier phoA-Gen) integriert haben.

Heffron et al. haben für die partielle Spaltung Pankreas-Desoxyribonuklease verwendet. In gleicher Weise kann man auch Restriktionsendonukleasen benutzen, die sehr häufig und daher auch mit hoher Wahrscheinlichkeit in dem zu verändernden Gen schneiden. Dies sind vor allem die Restriktionsendonukleasen, welche Sequenzen von 4 Basenpaaren erkennen. Sie haben häufig den zusätzlichen Vorteil gegenüber Pankreas-DNAse, daß sie direkt komplette Doppelstrangenden liefern. Eine aktuelle Übersicht über die bekannten Restriktionsendonukleasen gibt z.B. R. I. Roberts (in: Methods in Enzymology, Vol. 68, S. 27; S. P. Colowick und N. O. Kaplan Ed.; Academic Press, New York 1979).

Die Anwendbarkeit dieses Verfahrens zur Konstruktion neuer Expressionsvektoren wird durch die Herstellung des Plasmids pSB90 (Beispiel 9) belegt, welches durch Einfügen eines synthetischen Oligonukleotids mit der Erkennungssequenz für die Restriktionsendonuklease BamHI in das phoA-Gen des Plasmids pSB60 erhalten wurde. pSB60 wurde aus pSB53 (vgl. Beispiel 4) durch Beseitigung der darin bereits vorhandenen BamHI-Sequenz hergestellt, damit pSB90 nur die eine neu eingeführte BamHI-Sequenz enthält. Für die partielle Spaltung von pSB60 wurde die Restriktionsendonuklease HaeIII verwendet. Verschiedene synthetisches Oligonukleotide, die für die gleiche Anwendung in Frage kommen, sind beschrieben worden (vgl. z.B. R. I. Rothstein et al., in: Methods in Enzymology Vol. 68, S. 98; S. P. Colowick und N. O. Kaplan Ed.; Academic Press, New York, 1979) und zum Teil kommerziell verfügbar.

Am 16. Juli 1979 wurden bei der American Type Culture Collection (ATCC) in Rockville, Md., USA folgende Pasmide und Plasmid-haltigen Bakterienstämme hinterlegt (Hinterlegungsnummer):

Plasmid pWB2 (ATCC 40013)
Plasmid pBR313 (ATCC 40014)
Plasmid pBR322 (ATCC 40015).
Plasmid pSB18 (ATCC 40016)
Plasmid pSB47 (ATCC 40017)
Plasmid pSB50 (ATCC 40018)
Plasmid pSB51 (ATCC 40019)
Plasmid pSB53 (ATCC 40020)
Plasmid pSB87 (ATCC 40021) und
*Escherichia coli* SB44 mit pSB47 (ATCC 31540)
*E. coli* SB44 mit pSB51 (ATCC 31541)
*E. coli* SB44 mit pSB53 (ATCC 31542)
*Serratia marcescens* Wildtyp mit pSB51 (ATCC 31543)

Am 16. Juli 1980 bei der "Deutschen Sammlung für Mikroorganismen (DSM)" hinterlegt:

*E. coli* SB44 mit pSB60 (DSM 1873)
*E. coli* SB44 mit pSB86 (DSM 1874)
*E. coli* SB44 mit pSB90 (DSM 1875)

Außerdem wurden am 16. Juli 1979 bei der Deutschen Sammlung von Mikroorganismen (DSM) in Göttingen, Bundesrepublik Deutschland, folgende Mikroorganismen hinterlegt (Hinterlegungsnummer):

*Escherichia coli* SB44 (DSM 1606)
*Escherichia coli* HB101 (DSM 1607)
*Serratia marcescens* Wildtyp (DSM 1608)

Für den Mikroorganismus *Escherichia coli* K12 Wildtyp liegt bereits seit dem 26.11.1973 eine Hinterlegung unter der DSM-Aufnahmenummer DSM 498 vor.

Beispiel 1
Isolierung der phoA-Mutante E. coli SB44

25 ml L-Medium, welches 10 g/l Tryptone (Difco), 5 g/l Yeast Extract und 5 g/l NaCl enthält [E. S. Lennox, Virology, *1*, 190—206 (1955)], wurden mit 0,25 ml einer Übernachtkultur von E. coli K12 HB101 [H. W. Boyer, D. Roulland-Dussoix, J. Mol. Biol. *41*, 459—472 (1969)] in L-Medium beimpft und in einem temperierbaren Schüttler bei 37°C inkubiert. Bei Erreichen der exponentiellen Wachstumsphase ($E_{500}$ ca. 1,5) wurden aus der Kultur Proben von je 5 ml entnommen und mit je 0,1 ml einer entsprechenden Verdünnung einer frisch bereiteten Lösung von 5 g/l 1-Nitroso-3-nitro-1-methylguanidin (NNMG) versetzt, so daß Endkonzentrationen von 0 (Kontrolle), 10, 25, 50 und 100 µg/ml NNMG entstanden. Die Proben wurden 10 Minuten bei 37° inkubiert, sofort danach bei +4°C abzentrifugiert, zweimal mit je 5 ml kalter steriler NaCl-Lösung (9 g/l) gewaschen und in je 5 ml NaCl-Lösung resuspendiert. Durch Ausplattieren von Verdünnungsreihen auf Agarplatten (1,5% Agar in L-Medium) wurden die Lebendkeimzahlen ermittelt.

Vergleich mit dem Wert der Kontrolprobe ohne NNMG zeigte, daß der Mutationsansatz mit 10 µg/ml NNMG eine für die Mutantenselektion besonders günstige Überlebensrate von 0,5% hatte.

Aus einer Verdünnung diese Ansatzes, welche ca. 1.000 Zellen pro ml enthielt, wurden Proben von je 0,1 ml auf Agarplatten (0,15% Agar) mit LP-Medium [K. Kreuzer et al., Genetics *81*, 459—468 (1975)] ausplattiert, welches mit 20 mg/l L-Prolin und 20 mg/l L-Leucin supplementiert war. In diesem Medium ist die Synthese von alk. Phosphatase wegen des niedrigen Phosphatgehalts dereprmiert.

Nach einer Betrütung von 48 Stunden bei 37°C wurden die Platten, auf denen Einzelkolonien entstanden waren, mit einer Lösung von 5 mg/ml p-Nitrophenylphosphat in 1 M TrisHCl-Puffer, pH 8,0 überschichtet. Einzelkolonien, die nicht innerhalb weniger Minuten eine Gelbfärbung zeigten, wurden abgeimpft und durch quantitative Bestimmung der Bildung von alk. Phosphatase, wie in der Tabelle beschrieben, weiter geprüft.

Es wurden zwei phosphatasenegative Mutanten gefunden, die als E. coli SB43 und SB44 bezeichnet wurden. Wie in Beispiel 3 gezeigt wird, bewirkt das Plasmid pSB51 in der E. coli-Mutante SB44 die Bildung der alkalischen Phosphatase aus S. marcescens. Daher muß pSB51 einerseits ein phoA-Gen aus S. marcescens enthalten und die E. coli-Mutante SB44 muß ein phoA-Mutanten sein. Da die Mutante SB43 nach Transformation mit pSB51 nicht Phosphatase-positive wird, muß sie eine phoB-Mutante sein. Diese Zuordnung wurde bestätigt durch Transformation von authentischen phoA- und phoB-Mutanten mit den in folgenden Beispielen beschriebenen Plasmiden.

Beispiel 2
a) Herstellung des Plasmids pSB47

Als Vektor wurde das Plasmid pBR313 eingesetzt, welches Resistenz gegen Ampicillin und Tetracyclin bewirkt [F. Bolivar et al., Gene *2*, 75—93 (1977)]. Das Plasmid pBR313 wurde nach der dort angegebenen Methode isoliert (ATCC-Nr. 40014). Hochmolekulare chromosomale DNS aus E. coli K12-Wildtypbakterien wurde nach dem Phenolextraktionsverfahren von Saito et al. [Biochem. Biophys. Acta *72*, 619—629 (1963)], jedoch unter Auslassung der RNAse-Verdauung isoliert. Beide sorten von DNS wurden gegen DNS-Buffer (45 mM TrisHCl, 0,1 mM EDTA, pH 7,9) dialysiert und in dieser Form weiterverwendet. Die BamHI wurde ebenso wie T4-Ligase (100 Einheiten/ml) von der Firma Biolabs (Beverly, Maryland, USA) bezogen. Plasmid und chromosomale DNS wurden mit der Restriktionsendonuklease BamHI (von der Firma Biolabs bezogen, 4.000 Einheiten/ml) umgesetzt. Die BamHI-Reaktionsmischung hatte die folgende Zusammensetzung: 15 µl pBR313 (250 µg/ml DNS), 45 µl chrom. DNS aus E. coli K12 (180 µg/ml). 60 µl BamHI-reaktionspuffer (20 mM TrisHCl, 60 mM KCl, 20 mM MgCl$_2$, 20 mM Dithiothreitol, pH 7,5), 60 µl BamHI (1:40 verdünnt). Es wurde 60 Minuten bei 37°C inkubiert und zum Abstoppen der enzymatischen Reaktion 10 Minuten auf 65° erwärmt. Die für eine quantitative Umsetzung notwendige Enzymverdünnung war in einem Vorversuch ermittelt worden, wobei der Grad der Umwandling von ringförmiger in lineare Plasmid-DNS durch Gelelektrode in 0,7% Agarose [J. F. Morrow et al.l Proc. Natl. Acad. Sci USA *71*, 1743—1747 (1974)] bestimmt wurde.

Zu der obigen Reaktionsmischung (180 µl) wurden 40 µl T4-Ligase-Puffer (660 mM TrisHCl, 66 mM MgCl$_2$, 100 mM Dithiothreitol, pH 7,6), 40 µl ATP (4 mM), 2 µl T4-Ligase und 138 µl H$_2$O gegeben (Gesamtvolumen 400 µl). Die Mischung wurde 16 Stunden bei 13°C inkubiert, zweimal mit je 400 µl Phenol, welches zuvor mit TES-Puffer (50 mM TrisHCl, 50 mM NaCl, 5 mM EDTA, pH 8,0) gesättigt worden war, extrahiert und gegen DNS-Puffer phenolfrei dialysiert.

b) Transformation und Selektion von pho$^+$-Zellen

Mit der Ligaseumsetzung wurden kompetente Zellen der phoA-Mutante E. coli SB44 (Beispiel 1) transformiert.

Zur Herstellung der kompetenten Zellen wurden ein leicht modifiziertes Verfahren von Lederberg et al. [J. Bacteriol. *119*, 1072—1074 (1974)] angewendet. Die Zellen wurden wie in Beispiel 1 in L-Medium (100 ml) bis zur opt. Dichte E$_{500}$=0,8 angezüchtet, abzentrifugiert und in 100 ml MgCl$_2$-Lösung (100 mM) suspendiert. Nach 20 Minuten bei 0°C und Zentrifugation wurden die Zellen in 50 ml CaCl$_2$-Lösung (30 mM) aufgenommen und nach weiteren 20 Minuten bei 0°C abzentrifugiert, in 5 ml CaCl$_2$ (30 mM) suspendiert und bis zur Weiterverwendung in Eis aufbewahrt.

Zur Transformation/W. Goebel und R. Bonewald, J. Bacteriol. *123*, 658—665 (1975)/wurden 200 µl eisgekühlte Ligaseumsetzung und 400 µl kompetente Zellen gemischt, 50 Sek. im 38°C-Wasserbad erwärmt und 60 Minuten bei 0°C aufbewahrt. Nach Zugabe von 8 ml L-Medium wurden die Zellen 2 Stunden bei 37° inkubiert, dann in L-Medium (100 ml) mit 50 µg/ml Ampicillin überführt und weitere 16 Stunden bei 37°C inkubiert. Aus dieser Ampicillinanreicherung wurden phosphatasepositive Einzelkolonien mit der gleichen Methode isoliert, die in Beispiel 1 zur Isolierung von phosphatasenegativen Mutanten verwendet wurde. Unter ca. 10.000 farblosen Kolonien fand sich jeweils eine gelbe.

c) Isolierung und Charakterisierung von pSB47

Aus den gelben Kolonien ließ sich nach bekannten Verfahren [G. O. Humphreys et al., Biochem. Biophys. Acta *383*, 457—463 (1975)] ein Plasmid vom MG 16,8 Md isolieren, welches als pSB47 bezeichnet wurde und welches nach Schneiden mit BamHI in Agarosegel 2 DNS-Banden vom MG 5,8 Md (lineares pBR313) und 11 Md (chromosomales pho-Fragment) zeigte. Durch Transformation der in Beispiel 1 genannten Mutanten wurde gezeigt, daß pSB47 ein phoA-Gen enthält.

Beispiel 3

Herstellung des Plasmids pSB51

Das Vektorplasmid pWB2, welches eine Schnittstelle für die Restriktionsendonuklease HindIII besitzt und Resistenz gegen Ampicillin und Colicin EI vermittelt [W. Biodol et al., Molec. Gen. Genet. 152, 231—237 (1977)] wurde nach literaturbekannten Verfahren [G. O. Humphreys et al., Biochem. Biophys. Acta *383*, 457—463 (1975)] isoliert. Das Verfahren unterscheidet sich von dem in Beispiel 2 beschriebenen dadurch, daß anstelle von E. coli-DNS 45 µl chromosomale DNS (135 µg/ml) aus Serratia marcescens Wildtyp eingesetzt wurde und daß der Vektor pWB2 und die DNS aus Serratia marcescens mit der Restriktionsendonuklease HindIII geschnitten wurden. Eine Rk-Mischung, bestehend aus 15 µl pWB2 (360 µg/ml DNS), 45 µl chromosomaler DNS (135 µg/ml), 60 µl HindIII-Reaktionspuffer (20 mM Tris-HCl, 20 mM Mercaptoäthanol, 20 mM $MgCl_2$, 180 mM NaCl, pH 7,4) und 60 µl einer verdünnten HindIII-Lösung, wurde wie in Beispiel 2 60 Minuten bei 37°C inkubiert und zum Abstoppen der enzymatischen Reaktion 10 Minuten auf 65°C erwärmt. Weitere Umsetzungen analog Beispiel 2. Aus den wie in Beispiel 2 isolierten phosphatasepositiven Kolonien ließ sich ein als pSB51 bezeichnetes Plasmid vom MG 9,6 Md isolieren, welches nach Umsetzung mit HindIII in der Gelelektrophorese 2 DNS-Banden vom MG 7,1 Md (lineares pWB2) und 2,5 Md (chromosomales Fragment) aufwies. Durch Transformation ließ sich wie in Beispiel 2 zeigen, daß pSB51 nur ein phoA-Gen enthält.

Die von E. coli SB44 (pSB51) produzierte alkalische Phosphatase wurde durch Disk-Elektrophorese mit den entsprechenden Enzymen aus E. coli K12 und S. marcescens verglichen. Dazu werden die Rohenzyme durch osmotischen Schock aus den induzierten Zellen isoliert [A. Torriani, in Procedures in Nucleic Acid Research, S.224—235, G. L. Cantoni und D. R. Davies, ed. Harper und Row, Publishers, New York 1966].

In einzelnen wurden die Zellen (ca. 1,5 g Feuchtgewicht) auf 0,5 l einer wie in der Tabelle hergestellten Schüttelkultur durch Zentrifugations geerntet, dreimal mit 10 mM TrisHCl, pH 7,7 gewaschen und in 30 ml Rohrzuckerlösung (0,5 M Saccharose, 30 mM TrisHCl, 0,5 mM EDTA, pH 8,0) suspendiert. Die Suspension wurde 10 Minuten bei Raumtemperatur gerührt. Die Zellen wurden abzentrifugiert, in 30 ml Wasser von 3—5°C suspendiert, 10 Minuten gerührt und erneut abzentrifugiert. Aus dem Überstand wurde das Rohenzym mit $(NH_4)_2 SO_4$ (85% Sättigung) gefällt, der Proteinniederschlag wurde in 0,6 ml 37 mM TrisHCl pH 8,5 gelöst und gegen den gleichen Puffer dialysiert.

Zur Disk-Elektrophorese [H. R. Maurer, Disk-Elektrophorese, Verlag Walter de Gruyter, Berlin 1968] der rohen Phosphatasen wurden Acrylamid-Flachgele, bestehend aus 10%igen Trenngel in 0,185 M TrisHCl, pH 8,5 und 5%igen Sammelgel in 0,037 M TrisHCl, pH 8,5 mit 0,037 M Tris-Borat, pH 8,5 als Elektrophoresepuffer verwendet. Die Trennung erfolgte bei 450V (4 Std.). Die Phosphatasebanden wurden im Gel durch eine spezifische Farbreaktion [O. Gabriel, in Methods in Enzymol., Vol. 22, S. 590, W. B. Jakoby, ed., Academic Press 1971] nachgewiesen, welche auf der Hydrolyse von α-Naphtholphosphat zu α-Naphthol und Bildung eines Azofarbstoffes beruht.

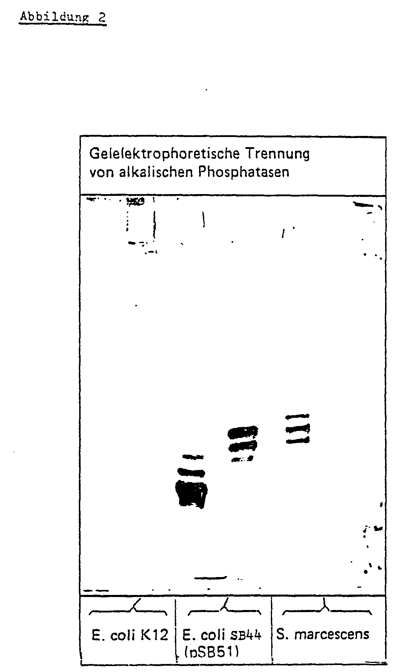

Die Enzympräparationen aus den beiden Referenzstämmen ergaben die bekannten Muster von je 3 dicht beieinander liegenden Phosphatasebanden, die sich eindeutig unterscheiden (die Banden des Enzyms aus S. marcescens laufen merklich langsamer als diejenigen des E. coli K12-Enzyms). Das Enzym aus E. coli SB44 (pSB51) zeigte ein Bandenmuster, welches mit dem Muster des S. marcescens-Enzyms übereinstimmte (s. Abbildung 2).

Beispiel 4

Herstellung des Plasmids pSB53

a) Herstellung des Plasmids pSB18

pSB18 wurde aus dem Plasmid pBR322 durch Entfernen der EcoRI-Restriktionssequenz hergestellt. pBR322 ist von Bolivar et al. [Gene *2*, 95—113 (1977)] beschrieben worden und wurde wie dort angegeben isoliert. Die DNS wurde als Lösung in 45 mM TrisHCl, 0,1 mM EDTA, pH 7,9 (DNS-Puffer) weiterverwendet.

Die Restriktionsendonuklease EcoRI wurde nach literaturbekannten Verfahren (P. J. Greene et al. in Methods in Molecular Biology Vol. 7, S.7, R. B. Wickner ed., Marcel Dekker Inc., New York, 1974) isoliert. T4-DNS-Polymerase (5.000 Einheiten/ml) wurde von der Firma PL-Biochemicals Inc. (Milwaukee, Wisl. USA) und T4-DNS-Ligase (100 Einheiten/ml) von der Firma Biolabs (Beverly, Maryland, USA) bezogen.

Eine Reaktionsmischung, bestehend aus 10 µl pBR322 (620 µg/ml), 10 µl EcoRI-Reaktionspuffer (300 mM TrisHCl, 30 mM $MgCl_2$, pH 7,5) und 10 µl einer verdünnten EcoRI-Lösung wurde 60 Minuten bei 37°C inkubiert und zum Abstoppen der enzymatischen Reaktion 10 Minuten auf 65°C erwärmt. Die für eine quantitative Umsetzung notwendige Enzymverdünnung war in einem Vorversuch ermittelt worden, wobei der Grad der Umwandlung von ringförmiger in lineare Plasmids-DNS durch Gelelektrophorese in 0,7% Agarose [J. F. Morrow et al. in Proc. Natl. Acad. Sci. USA *71*, 1743—1747 (1974)] bestimmt wurde.

Zu dem Ansatz (30 µl Volumen) wurden 10 µl Polymerase-Puffer (600 mM TrisHCl, 80 mM $MgCl_2$, 100 mM Mercaptoäthanol, pH 7,5) je 10 µl von 500 µM Lösungen von 2'-Desoxy-adenosin-5'-triphosphat (dATP), 2'-Desoxy-guanosin-5'-triphosphat (dGTP), 2'-Desoxy-thymidin-5'-triphosphate (dTTP) und 2'-Desoxy-cytidin-5'-triphosphate (dCTP) sowie 10 µl T4-DNS-Polymerase (1;2000 verdünnt) und 10 µl Wasser zugegeben. Die Mischung (100 µl Volumen) wurde 30 Minuten auf 37°C und zum Abstoppen 10 Minuten auf 70°C erwärmt.

Danach wurden 15 µl Ligase-Puffer (660 mM TrisHCl, 66 mM $MgCl_2$, 100 mM Dithiothreitol, pH 7,6), 15 µl ATP (4 mM), 2 µl T4-DNS-Ligase und 10 µl H$_2$O zugefügt. Die Mischung (15) µl Volumen) wurde 16 Stunden bei 13°C inkubiert und 1 Minuten auf 65°C erwärmt.

Um restliches pBR322 in die schleckt transformierende lineare Form umzuwandeln, wurde die Mischung noch einmal wie oben mit EcoRI umgesetzt (Reaktionsvolumen 250 µl), anschließend zweimal mit je 250 µl Phenol, welches zuvor mit DNS-Puffer gesättigt worden, war, extrahiert und gegen DNS-Puffer phenolfrei dialysiert (300 µl Endvolumen).

Von dem Stamm E. coli K12 HB101 [H. W. Boyer, D, Roulland-Dussoix, J. Mol. Biol. *41*, 459—472 (1969)] wurden kompetente Zellen hergestellt. Zur Herstellung der kompetenten Zellen wurde ein leicht modifiziertes Verfahren von Lederberg et al. [J. Bacteriol. *119*, 1072—1074 (1974)] angewendet. Die Zellen wurden wie in Beispiel 1 in L-Medium (100 ml) bis zur opt. Dichte E$_{500}$=0,8 angezüchtet, abzentrifugiert und in 100 ml MgCl$_2$-Lösung (100 mM) suspendiert. Nach 20 Minuten bei 0°C und Zentrifugation wurden die Zellen in 50 ml CaCl$_2$-Lösung (30 mM) aufgenommen und nach weiteren 20 Minuten bei 0°C abzentrifugiert, in 5 ml CaCl$_2$ (30 mM) suspendiert und bis sue Weiterverwendung in Eis aufbewahrt. Zur Transformation wurden 200 µl der nach Ligaseumsetzung erhaltenen DNS-Lösung und 400 µl kompetente Zellen gemischt, 50 Sekunden in 38°C-Wasserbad erwärmt und 60 Minuten bei 0°C aufbewahrt. Nach Zugabe von 8 ml L-Medium (10 g/l Tryptone (Difco), 5 g/l Yeast Extrakt, 5 g/l NaCl) [E. S. Lennox, Virology *1*, 190—206 (1955)] wurden die Zellen 2 Stunden bei 37°C inkubiert, dann in L-Medium (100 ml) mit 50 µl/ml Ampicillin überführt und weitere 16 Stunden bei 37°C inkubiert. Aus dieser Ampicillinanreicherung wurden in üblicher Weise auf Agarplatten Einzelkolonien isoliert, die resistent gegen Ampicillin (100 µg/ml) und Tetracyclin (25 µg/ml) waren. Aus 4 Einzelkolonien wurde nach bekannten Verfahren [G. O. Humphreys et al., Biochem. Biophys. Acta *383*, 457—463 (1975)] Plasmid-DNS isoliert. In allen 4 Fällen wurde ein Plasmid isoliert, welches sich von dem Ausgangsplasmid pBR322 nur durch das Fehlen der Restriktionssequenz für EcoRI unterschied. Es erhielt die Bezeichnung pSB18.

b) Herstellung des Plasmids pSB53

Die Isolierung der Restriktionsendonuklease HindIII erfolgte nach bekannten Verfahren [H. O. Smith, K. W. Wilcox, J. Mol. Biol. *51*, 379—391 (1970) und P. Philippsen, R. E. Streek, H. G. Zachau, Eur. J. Biochem. *45*, 479—488 (1974)]. Die Restriktionsendonuklease BamHI wurde von der Firma Biolabs bezogen.

Eine Reaktionsmischung bestehend aus 2 µl pSB18 (350 µg/ml), 8 µl pSB47 (40 µg/ml), 10 µl HindIII-Reaktionspuffer (20 mM TrisHCl, 20 mM Mercaptoäthanol, 20 mM MgCl$_2$, 180 mM NaCl, pH 7,4), 5 µl verdünnter HindIII-Lösung und 5 µl BamHI-Lösung wurde 60 Minuten auf 37°C und 10 Minuten auf 65°C erwärmt. Die zur quantitativen Spaltung notwendigen Enzymverdünnungen waren zuvor wie in Beispiel 1 durch Gelelektrophorese ermittelt worden.

Zu der Mischung wurden 10 µl Ligase-Puffer, 10 µl ATP (4 mM), 1 µl T4-Ligase und 50 µl H$_2$O gegeben. Dann wurde 16 Stunden bei 13°C inkubiert, zweimal mit Phenol extrahiert und gegen DNS-Puffer dialysiert.

Die Transformation der phosphatase-negativen Mutante SB44 mit der DNS-Lösung und die Selektion von ampicillinresistenten und phosphatase-positiven Einzelkolonien erfolgte wie in Beispiel 2b.

Aus einigen dieser Kolonien ließ sich ein als pSB53 bezeichnetes Plasmid vom Molekulargewicht 8600 Bp isolieren, welches durch gleichzeigige Spaltung mit den Restriktionsendonukleasen HindIII und BamHI in 2 Fragmente mide den Molekulargewichten 4020 Bp und 4600 Bp zerlegt wurde. Das größere Fragment erwies sich bei der Analyse durch Gelelektrophorese als identisch mit einem Fragment, welches sowohl bei der Doppelverdauung von pSB47 als auch von pSB47 mit HindIII und BamHI entsteht und welches daher das Gen für alkalische Phosphatase tragen muß. Das 2. Fragment aus pSB53 (4020 Bp) war identisch mit den aus pSB18 durch Doppelverdauung entstehenden größeren Fragment.

Beispiel 5
Eigenschaften von pSB53

Zur Herstellung einer Restriktionskarte wurde das Plasmid pSB53 mit den Restriktionsendonukleasen EcoRI, HindIII, BamHI, PstI, SalI, BglII, KpnI, XhoI und XbaI umgesetzt. EcoRI, HindIII und BamHI waren die gleichen Chargen wie im Beispiel 1, die übrigen Enzyme wurden von der Firma Biolabs bezogen. Die Standardreaktionsmischungen enthielten 10 µl pSB53 (170 µg/ml), 10 µl Reaktionspuffer und 10 µl Enzymverdünnung. Es wurden Reaktionspuffer wie in Beispiel 4 bzw. wie vom Enzymhersteller angegeben verwendet. Die zur quantitativen Umsetzung notwendigen Enzymverdünnungen wurden in Vorversuchen durch Spaltung von DNS des Bakteriophagen λ emittelt. Die Reaktionsmischungen wurden 60 Minuten bei 37°C und nach Zugabe von 10 µl Stopplösung weitere 30 Minuten bei 37°C inkubiert. Die Stopplösung enthielt 100 mM EDTA, 400 mg/ml Saccharose und 0,5 mg/ml Proteinase K (Fa. Merck, Darmstadt). Die Reaktionsprodukte wurden durch Elektrophorese in Flachgelen aus 0,7% Agarose [J. F. Morrow et al. in Proc. Natl. Acad. Sci. USA *71*, 1743—1747 (1974)] oder 3,5% Polyacrylamid [T. Maniatis et al., Biochemistry 14, 3787—3794 (1975)] analysiert. Die Bestimmung der Molekulargewichte der DNS-Fragmente erfolgte durch Vergleich mit Eichfragmenten, die nach J. G. Sutcliffe [Nucl. Acids Res. *5*, 2721—2728 (1978)] aus pBR322 erhalten worden waren (Angaben in Basenpaaren, Bp).

Die Enzyme HindIII, BamHI, SalI und XhoI ergaben jeweils eine lineare Bande gleichen Molekulargewichts, während durch EcoRI und PstI zwei bzw. drei kleinere Banden entstanden. BglII, KpnI und XbaI spalteten pSB53 nicht.

Von den wie in Beispiel 4b durch gleichzeitige Spaltung mit HindIII und BamHI erhaltenen Banden hatte

diejenige vom Mg 4020 Bp die aus der Struktur von pBR322 bekannten Schnittstellen für Pstl und Sall, während die aus chromosomaler DNS von E. coli K12 abgeleitete Bande vom MG 4600 Bp durch Xhol in 2 Fragmente von 2580 Bp und 2020 Bp zerlegt wurde. Das Fragment mit 2580 Bp zerfiel mit EcoRi in drei Subfragmente (1210, 1040 und 330 Bp), während das Fragment mit 2020 Bp mit Pstl drei Subfragmente (820, 740 und 460 Bp) ergab. Aus diesen Daten sowie aus zusätzlichen vergleichenden Doppelumsetzungen mit HindlII/EcoRI und BamHI/Pstl ließ sich die in Abb. 1 dargestellte Restriktionskarte für pSB53 ableiten.

Zum Nachweis der Lage des phoA-Gens im Bereich der EcoRi-Schnittstellen von pSB53 wurde das EcoRI-Fragment mit 330 Bp durch ein anderes EcoRI-Fragment mit 3600 Bp ersetzt, welches das proA-Gen aus E. coli K12 trägt. Dieses Fragment kann aus dem Plasmid pSB37 erhalten werden, in dem es als Hybridplasmid mit pBR313 [F. Bolivar et al., Gene *2*, 75—93 (1977)] vorliegt. Durch Aufnahme von pSB37 werden der prolinbedürftige Stamm E. coli K12 HB101 und die davon abgeleitete phosphatasenegative Mutante E. coli SB44 prolinprototroph sowie ampicillin- und tetracylinresistent, während pSB53 nur Resistenz gegen Ampicillin bewirkt.

Eine Mischung aus 5 µl pSB53 (170 µg/ml), 5 µl pSB37 (µg/ml), 10 µl Reaktionspuffer und 10 µl EcoRI-Lösung wurde wie in Beispiel 4a umgesetzt und durch Erhitzen gestoppt. Die weitere Umsetzung der DNS-Mischung mit T4-Ligase, Transformation des Stammes E. coli SB44 und die Anreicherung von ampicillinresistenten Zellen erfolgte wie in Beispiel 4b. Die Zellen aus 2 ml dieser Kulturbrühe wurden abzentrifugiert, in 2 ml steriler NaCl-Lösung (9 g/l) suspendiert, mit NaCl-Lösung verdünnt und in verschiedenen Verdünnungsstufen auf Agarplatten mit Minimalmedium ohne Prolin (1,0 g/l $NH_4Cl$, 0,25 g/l $MgSO_4 \cdot 7H_2O$, 3,0 g/l $KH_2PO_4$, 7,0 g/l $Na_2HPO_4 \cdot 2H_2O$, 4,0 g/l Glucose, 0,5 G/l NaCl, 20 mg/l Leucin, 1 mg/l Vitamin B1, ph 7,0 (M9-Medium)) ausplattiert und bei 37°C zum Anwachsen von Einzelkolonien inkubiert. Tetracyclinsensitive Kolonien wurden durch überführen mit Hilfe der Stempeltechnik auf Agarplatten mit 10 µg/ml Tetracyclin identifiziert. Diese wurden auf gie Fähigkeit zur Bildung von alkalischer Phosphatase geprüft und erwiesen sich alle als phosphatasenegativ.

Die Plasmid-DNS, die sich aus den Kolonien mit dem Phänotyp $Pro^+$, $Pho^-$, $Ap^+$, $Tc^-$ isolieren ließ und welche die Bezeichnung pSB54 erhielt, wurde wie oben mit EcoRI gespalten und durch Gelelektrophorese analysiert. Es ließen sich zwei DNS-Banden vom Molekulargewicht 8290 Bp und 3600 Bp nachweisen, welche mit den entsprechenden Banden aus pSB53 und pSB57 identisch waren. Das Fragment mit 330 Bp aus pSB53 war dagegen in pSB54 nicht mehr vorhanden.

Beispiel 6

Herstellung der Plasmide pSB86 und pSB87

5. Reaktionsmischungen, bestehend aus je 5 µl pSB53 (170 µg/ml), 5 µl Wasser, 10 µl EcoRI-reaktionspuffer und 10 µl verschiedener EcoRI-Verdünnungen, wurden 60 Minuten bei 37°C inkubiert und zum Abstoppen der Reaktion 10 Minuten auf 65°C erhitzt. Es wurden Enzymverdünnungen von 1:200, 1:250, 1:320, 1:400 und 1:500 verwendet. In einem Vorversuch war ermittelt worden, daß die Verdünnung 1:200 gerade noch zur quantitativen Spaltung ausreicht, während die übrigen Verdünnungen nur partiell umsetzten.

Zu den Reaktionsmischungen von je 30 µl wurden je 20 µl Pstl-Reaktionspuffer (30 mM TrisHCl, 150 mM NaCl, 30 mM $MgCl_2$, pH 7,0) sowie je 10 µl Pstl-Lösung (Biolabs, 1:20 verdünnt) zugefügt. In einem Vorversuch war festgestellt worden, daß die Pstl-Menge zur quantitativen Umsetzung ausreicht. Die Mischungen wurden 60 Minuten bei 37°C inkubiert, anschließend zum Abstoppen der Reaktion mit je 15 µl einer Lösung aus EDTA (100 mM) und Saccharose (400 mg/ml) versetzt und durch Elektrophorese in Gelen aus 0,7% Agarose (vgl. Beispiel 5) aufgetrennt.

Während die Reaktionsmischung mit der EcoRI-Verdünnung 1:200 fünf DNS-Banden mit den Molekulargewichten 3980, 2030, 1827, 460 und 330 Bp aufwies, enthielten die übrigen Mischungen zusätzlich zwei bzw. drei Banden mit 2157, 2360 und 4180 Bp.

Die Doppelbande aus 2157 und 2360 Bp wurde durch elektrophoretische Adsorptions an Hydroxylapatit nach der Methode von H. F. Tabak und R. A. Flavell, Nucleic Acid Res. *5*, 2321—2332 (1978) aus dem Gel isoliert. Anschließend wurde die DNS vom Hydroxylapatit mit 1 M Natriumphosphatpuffer eluiert, dialysiert, mit 2 Vol. Äthanol gefällt und in DNS-Puffer gelöst.

Das Plasmid pBR322 wurde auf gleiche Weise mit EcoRI und Pstl umgesetzt, jedoch nur unter Bedingungen, die zur quantitativen Spaltung führten. Von den dabei entstehenden zwei DNS-Fragmenten wurde das größere (3610 Bp) wie oben durch elektrophoretische Adsorption an Hydroxylapatit isoliert.

Eine Mischung der so aus pSB53 und pBR322 isolierten Fragmente wurde wie in Beispiel 4b mit T4-Ligase umgesetzt. Mit dem Reaktionsprodukt wurde der phosphatasenegative Stamm E. coli SB44 transformiert, aus der Transformationsmischung wurden in L-Medium mit 10 µg/ml Tetracyclin resistente Zellen angereichert.

Tetracyclinresistente Einzelkolonien wurden durch Stempeln auf L-Medium mit 50 µg/ml Ampicillin bzw. auf LP-Medium [K. Kreuzer et al., Genetics *81*, 459—468 (1975)] aus Ampicillinresistenz und Phosphatasebildung geprüft. Alle Zellen waren phosphatasenegativ, ca. 70% waren ampicillinresistent und 30%-sensitiv.

Aus dem ampicillinresistenten Zellen ließ sich mit herkömmlichen Methoden ein als pSB86 bezeichnetes Plasmid isolieren, welches durch Schneiden mit EcoRI und Pstl drei Banden von 3610 Bp (aus pBR322) sowie 1827 und 330 Bp (aus pSB53) ergab. Aus den ampicillinsensitiven Zellen wurde pSB87

isoliert, welches mit EcoRI und PstI drei Banden von 3610, 2030 und 330 Bp ergab. Die Restriktionskarten von pSB86 und pSB87 sind in Abb. 1 dargestellt.

Beispiel 7
Herstellung des Plasmids pSB50
 a) In vitro DNS-Verknüpfung
 Das Vektorplasmid pWB2, welches eine Schittstelle für die Restriktionsendonuklease HindIII besitzt und Resistenz gegen Ampicillin und Colicin EI vermittelt [W. Boidol, G. Siewert, W. Lindenmaier, G. Luibrand, W. Goebel, Molec. Gen. Genet. *152*, 231—237 (1977)], wurde nach literaturbekannten Verfahren [G. O. Humphreys et al., Biochem. Biophys, Acta *383*, 457—463 (1975)] isoliert. Hochmolekulare chromosomale DNS aus E. coli K12-Wildtypbakterien wurde nach dem Phenolextraktionsverfahren von Saito et al. [Biochem. Biophys. Acta *72*, 619—629 (1963)], jedoch unter Auslassung der RNAse-Verdauung isoliert. Beide Sorten von DNS wurden gegen DNS-Puffer (45 mM TrisHCl, 0,1 mM EDTA, pH 7,9) dialysiert und in dieser Form weiterverwendet. Die Isolierung der Restriktionsendonuklease HindIII ist beschrieben worden·[J. Mol. Biol. *51*, 379—391 (1970) und Eur. J. Biochem. *45*, 479—488 (1974)]. T4-Ligase (100 Einheiten/ml) wurde von der Firma Biolabs (Beverly, Maryland, USA) bezogen.
 Eine Reaktionsmischung, bestehend aus 15 μl pWB2 (360 μg/ml DNS), 45 μl chromosomaler DNS (180 μg/ml), 60 μl HindIII-Reaktionspuffer (20 mM TrisHCl, 20 mM Mercaptoäthanol, 20 mM $MgCl_2$, 180 mM NaCl, pH 7,4) und 60 μl einer verdünnten HindIII-Lösung, wurde 60 Min. bei 37°C inkubiert und zum Abstoppen der enzymatischen Reaktion 10 Min. auf 65°C erwärmt. Die für eine quantitative Umsetzung notwendige Enzymverdünnung war in einem Vorversuch ermittelt worden, wobei der Grad der Umwandlung von ringförmiger in lineare Plasmid-DNS durch Gelelektrophorese in 0,7% Agarose [J. F. Morrow et al., Proc. Natl. Acad. Sci. USA *71*, 1743—1747 (1974)] bestimmt wurde.
 Zu der obigen Reaktionsmischung (180 μl) wurden 40 μl T4-Ligase-Puffer (660 mM TrisHCl, 66 mM $MgCl_2$, 100 mM Dithiothreitol, pH 7,6), 40 μl ATP (4 mM), 2 μl T4-Ligase und 138 μl $H_2O$ gegeben (Gesamtvolumen 400 μl). Die Mischung wurde 16 Stunden bei 13°C inkubiert, zweimal mit je 400 μl Phenol, welches zuvor mit TES-Puffer (50 mM TrisHCl, 50 mM NaCl, 5 mM EDTA, pH 8,0) gesättigt worden war, extrahiert und gegen DNS-Puffer phenolfrei dialysiert.

 b) Transformation und Selektion von pho⁺-Zellen
 Mit der Ligaseumsetzung wurden kompetente Zellen der phoA-Mutante E. coli SB44 (Beispiel 1) transformiert.
 Zur Herstellung der kompetenten Zellen wurde eine leicht modifiziertes Verfahren von Lederberg et al. [J. Bacteriol. *119*, 1072—1074 (1974)] angewendet. Die Zellen wurden wie in Beispiel 1 in L-Medium (100 ml) bis zur opt. Dichte $E_{500}$=0,8 angezüchtet, abzentrifugiert und in 100 ml $MgCl_2$-Lösung (100 mM) suspendiert. Nach 20 Min. bei 0°C und Zentrifugation wurden die Zellen in 50 ml $CaCl_2$-Lösung (30 mM) aufgenommen und nach weiteren 20 Min. bei 0°C abzentrifugiert, in 5 ml $CaCl_2$ (30 mM) suspendiert und bis zur Weiterverwendung in Eis aufbewahrt.
 Zur Transformation [W. Goebel und R. Bonewald in J. Bacteriol. *123*, 658—665 (1975)] wurden 200 μl eisgekühlte Ligaseumsetzung und 400 μl kompetente Zellen gemischt, 50 Sek. im 38°C-Wasserbad erwärmt und 60 Min. bei 0°C aufbewahrt. Nach Zugabe von 8 ml L-Medium wurden die Zellen 2 Stunden bei 37° inkubiert, dann in L-Medium (100 ml) mit 50 μg/ml Ampicillin überführt und weitere 16 Stunden bei 37°C inkubiert. Aus dieser Ampicillinanreicherung wurden phosphatasepositive Einzelkolonien mit der gleichen Methode isoliert, die in Beispiel 1 zur Isolierung von phosphatasenegativen Mutanten verwendet wurde. Unter ca. 10.000 farblosen Kolonien fand sich jeweils eine gelbe.

 c) Isolierung und Charakterisierung von pSB50
 Aus den gelben Kolonien ließ sich nach bekannten Verfahren [G. O. Humphreys et al., Biochem. Biophys. Acta *383*, 457—463 (1975)] ein Plasmid vom MG 22 Md isolieren, welches als pSB50 bezeichnet wurde. Dieses wurde wie oben mit der Restriktionsendonuklease HindIII umgesetzt und durch Gelelektrophorese in 0,7% Agarose [J. F. Morrow et al., Proc. Natl. Acad. Sci. USA *71*, 1743—1747 (1974)] analysiert. Es zeigten sich zwei DNS-Banden, von denen die schneller wandernde (MG 7,1 Md) mit linearen pWB2 identisch war. Die zweite Bande hatte ein Molekulargewicht von ca. 15 Md. Durch Transformation mit pSB50 wurden alle in Beispiel 1 genannten Mutanten phosphatasepositiv. pSB50 muß daher sowohl ein phoA- als auch ein phoB-Gen tragen, welche auf dem E. coli-Chromosom dicht benachbart sind.

Beispiel 8
Überführung von phoA-Plasmiden in Bakterienstämme
 Die in den Beispielen 2, 3, 4 und 7 beschriebenen Plasmide pSB47, pSB51, pSB53 und pSB50 wurden in E. coli SB44 und in die Phosphatasepositiven Stämme E. coli K12, E. coli HB101 und S. marcescens überführt. Dazu wurden die Zellen wie in Beispiel 2 kompetent gemacht und mit der isolierten DNS transformiert. Die so erhaltenen plasmidhaltigen Stämme sind in der Tabelle zusammengestellt, in welcher auch die Phosphataseproduktion unter dereprimierten Bedingungen angegeben und mit den entsprechenden Wertern der Ausgangsstämme verglichen werden.

Beispiel 9

Herstellung des Plasmids pSB90

a) Eliminierung der BamHI-Sequenz in pSB53

Es wurde die gleiche Reaktionsfolge angewendet wie bie der Herstellung von pSB18 (Beispiel 4a) mit dem Unterschied, daß als Ausgangsplasmid pSB53 eingesetzt und anstelle von EcoRI mit BamHI geschnitten wurde.

Eine Reaktionsmischung bestehend aus 10 µl pSB53 (490 µg/ml), 10 µl BamHI-Puffer (siehe Beispiel 2a) und 10 µl BamHI (1:40 verdünnt; von der Fa. Biolabs bezogen) wurde 60 Min. bei 37° inkubiert und zum Abstoppen 10 Min. auf 65°C erwärmt.

Zur weiteren Umsetzung mit DNS-Polymerase und T4-Ligase wurden die gleichen Reaktionskomponenten wie in Beispiel 4a unter den gleichen Bedingungen zugegeben.

Um restliches pSB53 in die schlecht transformierende lineare Form umzuwandeln,, wurde die Mischung noch einmal wie oben mit BamHI umgesetzt (Reaktionsvolumen 250 µl) und anschließend wie in Beispiel 4a mit Phenol extrahiert und dialysiert.

Die Transformation von E. coli SB44 und die Selektion von ampicillinresistenten und phosphatasepositiven Einzelkolonien erfolgte wie in Beispiel 2b. Aus diesem ließ sich ein als pSB60 bezeichnetes Plasmid isolieren, welches sich nur durch das Fehlen der Restriktrionssequenz für BamHI von pSB53 unterschied.

b) Einführung einer BamHI-Sequenz in das Gen für alkalische Phosphatase auf pSB60

Eine Reaktionsmischung, bestehend aus 50 µl pSB60 (440 µg/ml), 100 µl HaeIII-Reaktionspuffer (20 mM TrisHCl, 20 mM NaCl, 20 mM MgCl$_2$, 20 mM Mercaptoäthanol, 300 µg/ml Rinderserumalbumin, pH 7,4) und 150 µl einer 1:200 Verdünnung (2.85 Einheiten) der Restriktionsendonuklease HaeIII (Fa. Biolabs) wurde 60 Min. bei 37°C inkubiert, die Umsetzung wurde durch Zugabe von 100 µl Stopplösung (100 mM EDTA, 40% Saccharose) beendet. Die Enzymverdünnung war in einem Vorversuch wie in Beispiel 2a ermittelt worden und so bemessen, daß ca. 1/3 der ringförmigen Plasmid-DNS nur einmal geschnitten wurde und daher lineare Moleküle voller Länge ergab.

Die Reaktionsmischung wurde durch Elektrophorese in 0,7% Agarose-Gelen wie in Beispiel 5 aufgetrennt, die Bande mit der linearen DNS voller Länge wurde durch Vergleich mit HindIII geschittenem pSB60 identifiziert und aus dem Gel ausgeschnitten. Die DNS wurde nach bekannten Verfahren (H. O. Smith, in: Methods in Enzymology, Vol. 65, S 371 ff.; S. P. Colowick und N. O. Kaplan Ed.; Academic Press, New York 1980) durch Elektrophorese in einem Dialysierschlauch aus dem Gel eluiert und durch Phenolextraktion und Chromatographie aus DEAE-Cellulose gereinigt.

Eine Reaktionsmischung, bestehend aus 10 µl einer Lösung mit 100 µg/ml der synth. BamHI-Sequenz d(C-C-G-G-A-T-C-C-G-G) (Collaborative Research Inc., Waltham, Mass., USA), 3 µl Reaktionspuffer (660 mM TrisHCl, 66 mM MgCl$_2$, 100 mM Dithiothreitol, pH 7,6), 5 µl ATP-Lösung (4,0 mM), 7 µl Wasser und 5 µl Polynukleotid-Kinase (1.380 Einheiten/ml; Boehringer Mannheim) wurde 60 Min. bei 37°C inkubiert. Zu der Reaktionsmischung (30 µl) wurden 100 µl der durch Elektrophorese gereinigten linearen DNS (40 µg/ml), 15 µl ATP (4 mM), 15 µl Reaktionspuffer wie oben, 15 µl Wasser und 25 µl T4-Ligase (2,5 Einheiten; Fa. Biolabs) gegeben. Die Mischung (200 µl Gesamtvolumen) wurde 16 Std. bei 13°C inkubiert, mit Phenol extrahiert und gegen DNS-Puffer (45 mM Tris HCl, 0,1 mM EDTA, pH 7,9) dialysiert.

Die Lösung wurde wie in Beispiel 2 mit der Restriktionsendonuklease BamHI und darauf erneut mit T4-Ligase umgesetzt. Die Transformation der Mutante E. coli SB44 mit der DNS-Lösung, die Anreicherung von ampicillinresistenten Zellen und die Identifizierung von phosphatasennegativen Kolonien aus dieser Anreicherung erfolgte wie in Beispiel 2 bzw. Beispiel 1.

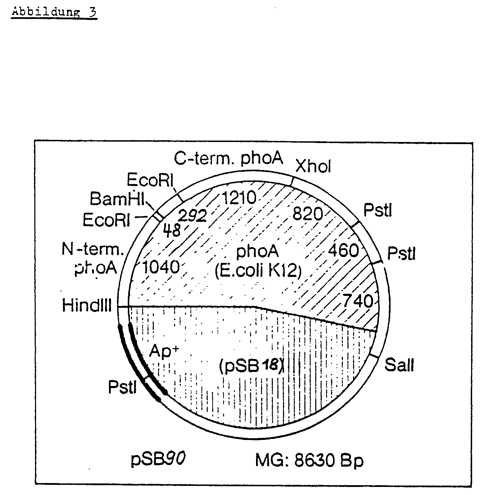

Aus einer ampicillanresistenten, phosphatasenegativen, Kolonie ließ sich ein als pSB90 bezeichnetes Plasmid isolieren. Die Charakterisierung von pSB90 erfolgte in ähnlicher Weise, wie in Beispiel 5 für pSB53 beschrieben, durch Umsetzung mit verschiedenen Restriktionsendonukleasen und Analyse der Reaktionsprodukte inklusive Molekulargewichtsbestimmung durch Gelelektrophorese. Abb. 3 zeigt die Restriktionskarte von pSB90, die aus diesen Daten abgeleitet wurde. Sie unterscheidet sich erwartungsgemäß von der Restriktionskarte für pSB53 (Abb. 1) nur durch die Lage der BamHI-Sequenz, die sich in pSB90 zwischen den beiden EcoRI-Sequenzen befindet, welche ebenfalls im Phosphatasegen liegen (vgl. Beispiel 5).

Zeichenerklärung zu Abbildung 1 und 3

Zur Bezeichnung von Erkennungssequenzen für Restriktionsenzyme wurden die folgenden Symbole verwendet:

| EcoRI
φ HindIII
Ỵ BamHI
† SalI
Ϥ PstI
ψ XhoI

Weitere verwendete Abkürzungen:

Ap$^+$ bzw. Ap$^-$—Ampicillinresistenz bzw.—sensitivität

**0 023 882**

Tc⁺ bzw. Tc⁻—Tetracylinresistenz bzw.—sensitivität
Pho⁺ bzw. Pho⁻—Fähigkeit zur Synthese von alk. Phosphatase vorhanden bzw. nicht vorhanden
Bp—Basenpaare.
Die in den Plasmiddarstellungen genannten Zahlen bedeuten Molekulargewichte in Bp.

## Patentansprüche

1. phoA⁻-Mutante E. coli SB44 (DSM 1606).

2 Verwendung der phoA⁻-Mutante E. coli SB44 gemäß Anspruch 1 zur Isolierung phoA-Gene enthaltender Plasmide.

3. Ein biologisch reiner Plasmidvektor, mit dem Bakterien zu Zellen mit 10—20 Kopien dieses Vektors pro Zelle transformiert werden können, enthaltend eine DNS-Sequenz für alkalische Phosphatase (phoA).

4. Plasmid pSB47 (ATCC 40017).

5. Plasmid pSB50 (ATCC 40018).

6. Plasmid pSB51 (ATCC 40019).

7. Plasmid pSB53 (ATCC 40020).

8. Plasmid pSB86 (DSM 1874).

9. Plasmid pSB60 (DSM 1873).

10. Plasmid pSB90 (DSM 1875).

11. Verfahren zur Herstellung von phoA-Gene enthaltenden Plasmiden, dadurch gekennzeichnet, daß man ein Antibiotikaresistentes Plasmid und chromosomale DNS aus Wildtyp-Bakterien mit Restriktionsendonukleasen umsetzt, anschliessend die Schnittstellen mit DNS Ligase verknüpft und unter Zuhilfenahme der aus E. coli K12 HB101 (DSM 1607) und 1-Nitroso-3-nitro-1-methyl-guanidin erhaltenen phoA⁻-Mutante E. coli SB 44 (DSM 1606) Plasmide mit Strukturgenen für die alkalische Phosphatase isoliert.

12. Verfahren zur Herstellung von phoA-Gene enthaltenden Plasmiden, dadurch gekennzeichnet, daß man aus den gemäß Anspruch 11 erhaltenen Plasmiden mit Hilfe von Restriktionsenzymen Subfragmente, die das phoA-Gen enthalten, herausschneidet und mit einem Antibiotika-resistenten Vektorplasmid mit Hilfe von DNS-Ligase rekombiniert.

13. Verfahren nach Anspruch 12 zur Herstellung des Plasmids pSB53 (ATCC 40020), dadurch gekennzeichnet, daß man das Antibiotika-resistente Vektorplasmid pSB18 (ATCC 40016) und eines der phoA-Plasmide pSB47 (ATCC 40017) oder pSB50 (ATCC 40018) mit den Restriktionsendonukleasen BamHI und HindIII schneidet.

14. Verfahren zur Herstellung des Plasmids pSB86 (DSM 1874), dadurch gekennzeichnet, daß man aus dem nach Anspruch 13 erhaltenen Plasmid pSB53 (ATCC 40020) durch vollständige Umsetzung mit PstI und nach anschließender partieller Spaltung mit EcoRI ein DNS-Fragment von 2157 Basenpaaren mit dem N-terminalen Ende des phoA-Gens isoliert und gemäß Anspruch 12 mit dem Vektorplasmid pBR322 (ATCC 40015) kuppelt.

15. E. coli-Stämme, enthaltend Plasmide mit einem Gen für alkalische Phosphatase nach Anspruch 3.

16. S. marcescens-Stämme, enthaltend Plasmide mit einem Gen für alkalische Phosphatase nach Anspruch 3.

17. Verfahren zur Herstellung neuer E. coli- und S. marcescens-Stämme, die Plasmide mit phoA-Genen enthalten, dadurch gekennzeichnet, daß man kompetente Zellen des betreffenden Stammes mit der Plasmid-DNS nach Anspruch 3 unter Transformationsbedingungen inkubiert und aus der Zellenpopulation Antibiotika-resistente Zellen isoliert.

18. Verwendung von E. coli. Stämmern, die ein Plasmid mit einem Gen für alkalische Phosphatase enthalten zur Herstellung der alkalischen Phosphatase.

19. Verwendung von S. marcescens-Stämmen, die ein Plasmid mit einem Gen für alkalische Phosphatase enthalten, zur Herstellung der alkalischen Phosphatase.

20. Verfahren zur Herstellung von alkalischer Phosphatase, dadurch gekennzeichnet, daß man die Stämme S. marcescens ATCC 31543 oder E. coli ATCC 31541 in einem Medium mit minimalen Phosphatgehalt fermentiert und die gebildete Phosphatase nach bekanntem Verfahren isoliert.

21. Verfahren zur Herstellung von alkalischer Phosphatase, dadurch gekennzeichnet, daß man E. coli ATCC 31540, E. coli ATCC 31542 oder E. coli DSM 1873 in einem Medium mit minimalen Phosphatgehalt fermentiert und die gebildete Phosphatase nach bekanntem Verfahren isoliert.

22. Verfahren zur Herstellung des Plasmids pSB60 (DSM 1873), dadurch gekennzeichnet, daß man aus dem nach Anspruch 13 erhaltenen Plasmid pSB53 (ATCC 40020) durch vollständige Umsetzung mit BamHI, Auffüllen der Einzelstrangenden mit DNS-Polymerase, Ringschluß mit T4-Ligase und Transformation von E. coli SB44 (DSM 1606) die BamHI-Sequenz entfernt.

## Revendications

1. Mutant phoA⁻ E. coli SB44 (DSM 1606).

2. Application du mutant phoA⁻ E. coli SB44 selon la revendication 1 pour l'isolement de plasmides contenant des gènes pour phoA.

15

# 0 023 882

3. Vecteur plasmidien biologiquement pur avec lequel il est possible de transformer des bactéries en cellules renfermant de 10 à 20 copies de ce vecteur par cellule, vecteur qui contient une séquence d'ADN pour la phosphatase alcaline (phoA).

4. Plasmide pSB47 (ATCC 40017).

5. Plasmide pSB50 (ATCC 40018).

6. Plasmide pSB51 (ACTT 40019).

7. Plasmide pSB53 (ATCC 40020).

8. Plasmide pSB86 (DSM 1874).

9. Plasmide pSB60 (DSM 1873).

10. Plasmide pSB90 (DSM 1875).

11. Procédé de préparation de plasmides contenant des gènes pour phoA, procédé caractérisé en ce qu'on fait réagir avec des endonucléases de restriction un plasmide résistant aux antibiotiques et une ADN chromosomique provenant de bactéries de type sauvage, puis, au moyen d'une ADN-ligase, on unit les endroits de coupure et, à l'aide du mutant phoA⁻ E. coli SB44 (DSM 1606), obtenn à partir de E. coli HB101 (DSM 1607) et nitroso-1-nitro-3-méthyl-1-guanidine, on isole des plasmides contenant des gènes de structure pour la phosphatase alcaline.

12. Procédé de préparation de plasmides contenant des gènes pour phoA, procédé caractérisé en ce qu'on extrait, à partir des plasmides obtenues selon la revendication 11, à l'aide d'enzymes de restriction, des sous-fragments contenant le gène pour phoA et on le recombine, à l'aide d'une ADN-ligase, avec un plasmide vecteur résistant aux antibiotiques.

13. Procédé selon la revendication 12 pour la préparation du plasmide pSB53 (ATCC 40020), procédé caractérisé en ce qu'on coupe le plasmide vecteur résistant aux antibiotiques pSB18 (ATCC 40016) et l'un des plasmides pour phoA pSB47 (ATCC 40017) et pSB50 (ATCC 40018) avec les endonucléases de restriction BamHI et HindIII.

14. Procédé de préparation du plasmide pSB86 (DSM 1874), procédé caractérisé en ce qu'on isole à partir du plasmide pSB53 (40020) obtenu selon l'exemple 13, par réaction complète avec PstI, puis coupure partielle avec EcoRI, un fragment d'ADN de 2157 paires de bases avec l'extrémité N-terminale du gène pour phoA, et on le combine, selon la revendication 12, avec le plasmide vecteur pBR322 (ATCC 40015).

15. Souches d'E. coli qui contiennent des plasmides ayant un gène pour la phosphatase alcaline, selon la revendication 3.

16. Souches de S. marcescens qui contiennent des plasmides ayant un gène pour la phosphatase alcaline, selon la revendication 3.

17. Procédé de préparation de nouvelles souches d'E. coli et de S. marcescens contenant des gènes pour phoA, procédé caractérisé en ce qu'on fait incuber des cellules compétentes de la souche correspondante avec l'ADN plasmidien selon la revendication 3, dans des conditions de transformation, et on isole de la population de cellules celles qui sont résistantes aux antibiotiques.

18. Application de souches d'E. coli qui contiennent un plasmide ayant un gène pour la phosphatase alcaline, à la préparation de la phosphatase alcaline.

19. Application de souches de S. marcescens qu contiennent un plasmide ayant un gène pour la phosphatase alcaline, à la préparation de la phosphatase alcaline.

20. Procédé de préparation de la phosphatase alcaline, procédé caractérisé en ce qu'on fait fermenter les souches de S. marcescens ATCC 31543 ou d'E. coli ATCC 31541 dans un milieu ayant une teneur minimale en phosphate et on isole, par un procédé connu, la phosphatase formée.

21. Procédé de préparation de la phosphatase alcaline, procédé caractérisé en ce qu'on fait fermenter E. coli ATCC 31540, E. coli ATCC 31542 ou E. coli DSM 1873 dans une milieu à teneur minimale en phosphate et on isole, par une méthode connue, la phosphatase formée.

22. Procédé de préparation du plasmide pSB60 (DSM 1873), procédé caractérisé en ce qu'on élimine la séquence BamHI du plasmide pSB53 (ATCC 40020) obtenu selon l'exemple 13, par réaction complète avec BamHI, remplissage des extrémités simple brin avec l'ADN-polymérase, cyclisation avec la T4-ligase et transformation d'E. coli SB44 (DSM 1606).

**Claims**

1. phoA⁻-mutant *E. coli* SB44 (DSM 1606).

2. Use of the phoA⁻-mutant *E. coli* SB44 according to claim 1 for isolating the phoA containing plasmid.

3. A biologically pure plasmid vector with which bacteria can be transformed to cells with 10—20 copies of this vector per cell, which contains a first DNA sequence coding for alkaline phosphatase (phoA).

4. Plasmid pSB47 (ATCC 40017).

5. Plasmid pSB50 (ATCC 40018).

6. Plasmid pSB51 (ATCC 40019).

7. Plasmid pSB53 (ATCC 40020).

8. Plasmid pSB86 (DSM 1874).

9. Plasmid pSB60 (DSM 1873).

10. Plasmid pSB90 (DSM 1875).

11. Process for the preparation of plasmids containing a phoA-gene characterised in that an antibiotic resistant plasmid and chromosomal DNA from wild type bacteria are reacted with a restriction endonuclease, the terminal sites are linked with DNA ligase and with the use of the phoA⁻-mutant *E. coli* SB44 (DSM 1606), obtained from E. coli K12 HB101 (DSM 1607) and 1-nitroso-3-nitro-1-methyl-guanidin, plasmids with structure genes for alkaline phosphatase are isolated.

12. Process for the preparation of plasmids containing a phoA-gene characterised in that subfragments containing the phoA gene are cut from the plasmid obtained according to claim 11, using a restriction enzyme and are recombined with an antibiotic resistant vector plasmid using DNA ligase.

13. Process according to claim 12 for the preparation of plasmid pSB53 (ATCC 40020) characterised in that the antibiotic resistant vector plasmid pSB18 (ATCC 40016) and one of phoA plasmid pSB47 (ATCC 40017) or pSB50 (ATCC 40018) are cut with restriction endonucleases BamHI and HindIII.

14. Process for the preparation of plasmid pSB86 (DSM 1874), characterised in that there is isolated from the plasmid pSB53 (ATCC 40020), a DNA fragment of 2157 base pairs with the N-terminal end of the phoA-gene, by reacting to completion with PstI and partial splitting with EcoRI and coupling with the vector plasmid pBR322 (ATCC 40015) according to claim 12.

15. *E. coli* strains, containing plasmids with a gene for alkaline phosphatase according to claim 3.

16. *S. marcescens* strains, containing plasmids with a gene for alkaline phosphatase according to claim 3.

17. Process for the preparation of novel *E. coli* and *S. marcescens* strains that contains plasmids with phoA-genes, characterised in that competent cells of the strains concerned are incubated with the plasmid DNA according to claim 3 under transformation conditions and antibiotic resistant cells are isolated from the cell population.

18. Use of *E. coli* strains, that contain a plasmid with a gene for alkaline phosphatase, for the preparation of alkaline phosphatase.

19. Use of *S. marcescens* strains that contain a plasmid with a gene for alkaline phosphatase, for the preparation of alkaline phosphatase.

20. Process for the preparation of alkaline phosphatase characterised in that the strain *S. marcescens* ATCC 31543 or *E. coli* ATCC 31541 is fermented in a medium with minimal phosphate content and the phosphatase so formed is isolated by known methods.

21. Process for the preparation of alkaline phosphatase characterised in that the strain *E. coli* ATCC 31540, *E. coli* ATCC 31542 or *E. coli* DSM 1873 is fermented in a medium with minimal phosphate content and the phosphatase so formed is isolated by known methods.

22. Process for the preparation of plasmid pSB60 (DSM 1873) characterised in that the BamHI sequence is removed from the plasmid pSB53 (ATCC 40020) obtained according to claim 13, by reaction to completion with BamHI, resupplying the single strands with DNA polymerase, ring closing with T4-ligase and transformation of *E. coli* SB44 (DSM 1606).

Abbildung 1

pSB53
MG 8620 Bp
Phänotyp
Ap⁺Tc⁻Pho⁺

pSB53    MG: 8620 Bp

pSB86
MG 5770 Bp
Phänotyp
Ap⁺Tc⁺Pho⁻

pSB87
MG 5970 Bp
Phänotyp
Ap⁻Tc⁺Pho⁻

1

Abbildung 2

Gelelektrophoretische Trennung
von alkalischen Phosphatasen

E. coli K12 | E. coli SB44 | S. marcescens
| (pSB51) |

Abbildung 3

pSB90  MG: 8630 Bp